(19) Europäisches Patentamt
European Patent Office
Office européen des brevets

(11) Veröffentlichungsnummer: **0 347 382 A2**

(12) **EUROPÄISCHE PATENTANMELDUNG**

(21) Anmeldenummer: **89810432.8**

(22) Anmeldetag: **07.06.89**

(51) Int. Cl.[4]: **C 07 D 231/16**
C 07 D 231/56, A 01 N 43/56

(30) Priorität: **16.06.88 CH 2322/88**
**06.10.88 CH 3719/88**

(43) Veröffentlichungstag der Anmeldung:
**20.12.89 Patentblatt 89/51**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Pissiotas, Georg, Dr.**
**Am Sonnenrain 71**
**D-7850 Lörrach (DE)**

**Moser, Hans, Dr.**
**Hauptstrasse 67B**
**CH-4312 Magden (CH)**

**Brunner, Hans-Georg, Dr.**
**Wannenstrasse 14**
**CH-4415 Lausen (CH)**

(54) **5-(Pyrazol-1-yl)-benzoesäure-thiolester mit herbizider Wirkung.**

(57) Neue 5-Pyrazol-1-yl)-benzoesäurethiolester der untenstehenden Formel I haben gute pre- und postemergente selektivherbizide Eigenschaften ferner hemmen sie das Pflanzenwachstum. Die Ester entsprechen der Formel I

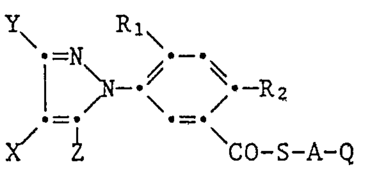

(I),

worin
X Wasserstoff, Halogen oder $C_1$-$C_4$-Alkyl,
Y $C_1$-$C_4$-Alkyl oder
X und Y gemeinsam mit den Kohlenstoffatomen an die sie gebunden sind einen 5-6-gliedrigen Ring bilden, der unsubstituiert oder durch Methyl substituiert ist,
Z Halogen, $C_1$-$C_6$-Alkyl, $C_1$-$C_4$-Alkoxy oder $C_1$-$C_4$-Alkylthio,
$R_1$ Wasserstoff oder Halogen,
$R_2$ Halogen,
A-Q gemeinsam Wasserstoff oder ein Rest -CH(COCH$_3$)CO-OR$_4$,
A eine $C_1$-$C_4$-Alkylenbrücke, und
Q, $R_3$ und $R_4$ einen in der Beschreibung definierten Rest verkörpert.

Es werden Synthesen zur Herstellung dieser Ester sowie Beispiele ihrer Verwendung beschrieben.

EP 0 347 382 A2

**Beschreibung**

**5-(Pyrazol-1-yl)-benzoesäure-thiolester mit herbizider Wirkung**

Die vorliegende Erfindung betrifft neue 5-Pyrazol-1-yl-benzoesäurethiolester der Formel I mit herbizider und den Pflanzenwuchs regulierender Wirkung, sowie die Herstellung dieser neuen Ester. Ferner betrifft sie Mittel, welche die neuen Verbindungen enthalten sowie deren Verwendung zur selektiven Bekämpfung von Unkräutern oder zur Regulierung des Pflanzenwuchses.

Die neuen 5-Pyrazol-1-yl-benzoesäurethiolester entsprechen der Formel I

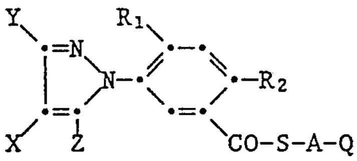

(I)

worin
X Wasserstoff oder Halogen
Y $C_1$-$C_4$-Alkyl oder
X und Y gemeinsam mit den Kohlenstoffatomen an die sie gebunden sind einen 5-6-gliedrigen Ring bilden, der unsubstituiert oder durch Methyl substituiert ist,
Z Halogen, $C_1$-$C_6$-Alkyl, $C_1$-$C_4$-Alkoxy oder $C_1$-$C_4$-Alkylthio
$R_1$ Wasserstoff oder Halogen,
$R_2$ Halogen,
A-Q gemeinsam Wasserstoff oder ein Rest -$CH(COCH_3)COOR_4$,
A eine geradkettige oder verzweigte $C_1$-$C_4$-Alkylenbrücke, die unsubstituiert oder ein- oder mehrfach durch $C_1$-$C_4$-Alkoxy, $C_1$-$C_4$-Alkylthio oder -CN substituiert ist,
Q Hydroxyl, Halogen, -CN, -SCN, $C_2$-$C_6$-Alkenyl, $C_2$-$C_6$-Haloalkenyl, $C_2$-$C_6$-Cyanoalkenyl, $C_2$-$C_4$-Alkinyl oder einen Rest -$C(R_3)=CH$-$COOR_4$, -$CH[N(R_3)_2]COOR_3$, -$NR_5R_6$, -$CONR_7R_8$, -$Si(R_{11})_3$, -$COOCH_2Si(CH_3)_2C_1$-$C_6$-alkyl, -$COON=C(R_9)_2$, -$C(R_3)=(OR_{10})_2$, -$PO(OR_{12})$-$(O)_pR_{12}$, -$CON(R_{13})SO_2$-$C_1$-$C_6$-alkyl, -$CON(R_{13})$-$SO_2C_1$-$C_4$-Haloalkyl, $C_1$-$C_6$-Alkylcarbonyl, $C_2$-$C_6$-Alkoxyalkylcarbonyl, Benzoyl oder Benzylcarbonyl, der unsubstituiert oder durch Halogen, $C_1$-$C_4$-Alkyl, $C_1$-$C_4$-Haloalkyl, $C_1$-$C_4$-Alkoxy, $C_1$-$C_4$-Haloalkoxy, Cyan oder Nitro ein- oder mehrfach substituiert ist, ferner einen Rest $COOR_{14}$, -$CON(R_3)CH_2C(OC_1$-$C_6$-Alkyl$)_2$ -oder $C_1$-$C_6$-Alkanoyloxyrest,
p ist Null oder 1,
$R_3$ Wasserstoff, $C_1$-$C_6$-Alkyl oder $C_2$-$C_6$-Alkoxyalkyl,
$R_4$ $C_1$-$C_6$-Alkyl oder $C_1$-$C_6$-Hydroxyalkyl,
$R_5$ und $R_6$ unabhängig voneinander je Wasserstoff, $C_1$-$C_6$-Alkyl, $C_2$-$C_6$-Alkoxyalkyl, $C_3$-$C_6$-Alkenyl, $C_3$-$C_6$-Alkinyl oder $C_3$-$C_6$-Cycloalkyl, 2-Furanylmethyl, 2-Tetrahydrofuranylmethyl, 2-(5-Methyl)-tetrahydrofuranylmethyl, 2-Thienylmethyl,
$R_5$ und $R_6$ zusammen mit dem Stickstoffatom an das sie gebunden sind, den Pyrrolidino-, Piperidino- oder Morpholinorest,
$R_7$ und $R_8$ unabhängig voneinander je Wasserstoff, $C_1$-$C_{12}$-Alkyl, $C_3$-$C_8$-Alkenyl, $C_3$-$C_6$-Alkinyl, $C_2$-$C_8$-Alkoxyalkyl, $C_1$-$C_4$-Alkoxy, Benzyl, Phenyl oder $C_1$-$C_4$-Cyanoalkyl,
$R_7$ und $R_8$ zusammen mit dem Stickstoffatom, an das sie gebunden sind, einen Pyrrolidino-, Piperidino-, Morpholino-, Thiomorpholino-, 4-Methylpiperazino-, Pyrazolino-, Imidazolino- oder 1,2,4-Triazolinrest der unsubstituiert oder ein- oder zweifach durch $C_1$-$C_4$-Alkyl substituiert ist,
$R_9$ unabhängig voneinander je $C_1$-$C_6$-Alkyl oder zusammen $C_3$-$C_7$-Cycloalkyl,
$R_{10}$ unabhängig voneinander $C_1$-$C_4$-Alkyl oder $C_1$-$C_4$-Haloalkyl oder zusammen eine 1,2-Aethylen-, 1,3-Propylen- oder 1,2-Cyclohexylenbrücke,
$R_{11}$ unabgängig voneinander $C_1$-$C_6$-Alkyl oder $C_1$-$C_6$-Alkoxy,
$R_{12}$ unabhängig voneinander Wasserstoff, $C_1$-$C_6$-Alkyl, $C_1$-$C_6$-Haloalkyl, $C_1$-$C_6$-Cyanoalkyl, $C_3$-$C_4$-Alkenyl oder $C_3$-$C_4$-Alkinyl,
$R_{13}$ $C_1$-$C_4$-Alkyl oder $C_3$-$C_7$-Cycloalkyl,
$R_{14}$ Wasserstoff, $C_1$-$C_{12}$-Alkyl, $C_1$-$C_6$-Alkoxy-$C_1$-$C_4$-alkyl, $C_3$-$C_7$-Alkenyl, $C_3$-$C_6$-Alkinyl, $C_3$-$C_7$-Cycloalkyl, $C_3$-$C_5$-Alkenyloxy-$C_1$-$C_4$-alkyl, $C_2$-$C_6$-Alkylthio-$C_1$-$C_4$-alkyl oder $C_1$-$C_4$-Dialkylamino-$C_1$-$C_4$-alkyl
bedeuten.

In den EP-A 207,894 und 233,151 sind ähnliche Tetrahydrophthalsäureimido-N-benzoesäureester beschrieben. Die vorliegenden Thiolester zeichnen sich denen gegenüber durch eine spezifischere Wirkung aus.

Speziell gute Wirkung zeigten die Verbindungen der untenstehenden Formeln, in denen Q eine der folgenden Bedeutungen hat:
- die Verbindungen der Formel Ia

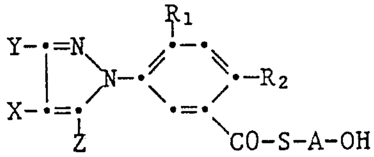

(Ia)

worin A, $R_1$, $R_2$, X, Y und Z die oben gegebene Bedeutung haben;
- die Verbindungen der Formel Ib

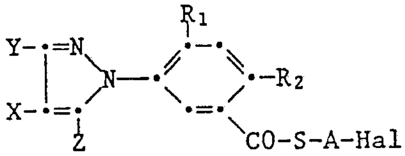

(Ib)

worin A, $R_1$, $R_2$, X, Y und Z die oben gegebene Bedeutung haben; und Hal ein Halogenatom bedeutet;
- die Verbindungen der Formel Ic

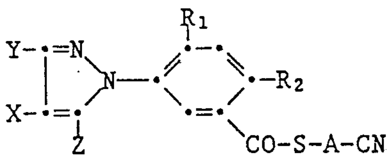

(Ic)

worin A, $R_1$, $R_2$, X, Y und Z die oben gegebene Bedeutung haben;
- die Verbindungen der Formel Id

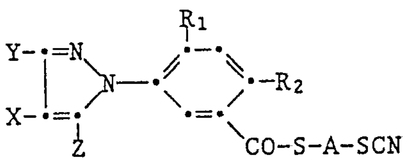

(Id)

worin A, $R_1$, $R_2$, X, Y und Z die oben gegebene Bedeutung haben;
- die Verbindungen der Formel Ie

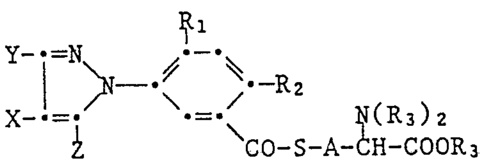

(Ie)

worin A, $R_1$, $R_2$ unabhängig voneinander die $R_3$, X, Y und Z die oben gegebene Bedeutung haben;
- die Verbindungen der Formel If

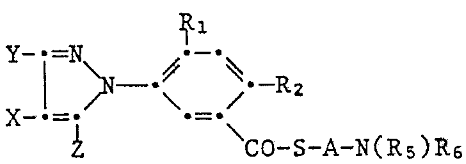

(If)

worin A, $R_1$, $R_2$, $R_5$, $R_6$, X, Y und Z die oben gegebene Bedeutung haben;
- die Verbindungen der Formel Ig

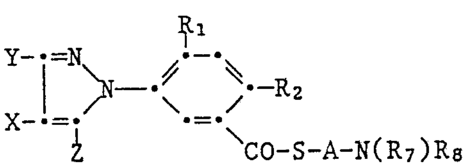

(Ig)

worin A, $R_1$, $R_2$, $R_7$, $R_8$, X, Y und Z die oben gegebene Bedeutung haben;
- die Verbindungen der Formel Ih

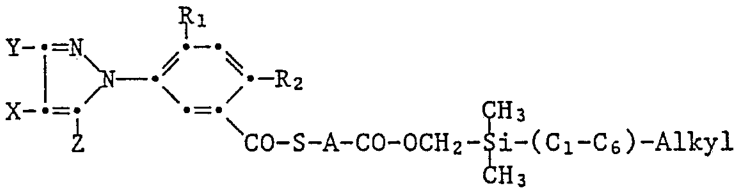

(Ih)

worin A, $R_1$, $R_2$, X, Y und Z die oben gegebene Bedeutung haben;
- die Verbindungen der Formel Ii

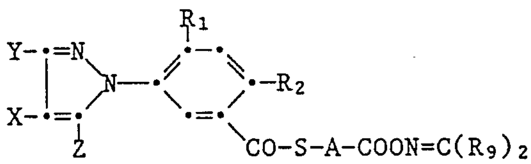

(Ii)

worin A, $R_1$, $R_2$, $R_9$, X, Y und Z die oben gegebene Bedeutung haben;
- die Verbindungen der Formel Ij

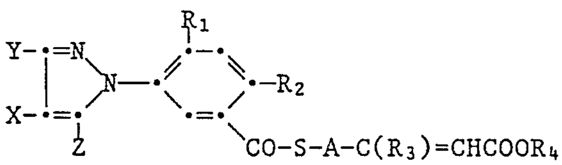

(Ij)

worin A, $R_1$, $R_2$, $R_3$, $R_4$, X, Y und Z die oben gegebenen Bedeutung haben:
- die Verbindungen der Formel Ik

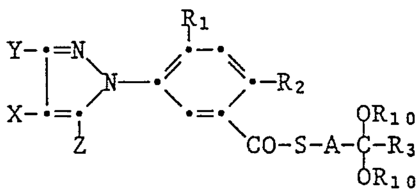

(Ik)

worin A, $R_1$, $R_2$, $R_3$, $R_{10}$, X, Y und Z die oben gegebenen Bedeutung haben;
- die Verbindungen der Formel Il

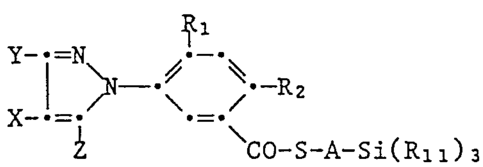

(Il)

worin A, $R_1$, $R_2$, $R_{11}$, X, Y und Z die oben gegebene Bedeutung haben;
- die Verbindungen der Formel Im

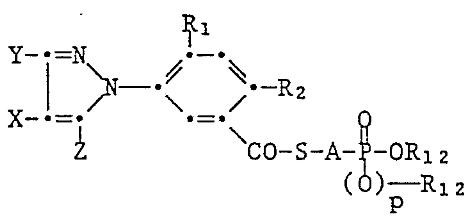

(Im)

worin A, p, $R_1$, $R_2$, $R_{12}$, X, Y und Z die oben gegebene Bedeutung haben;
- die Verbindungen der Formel In

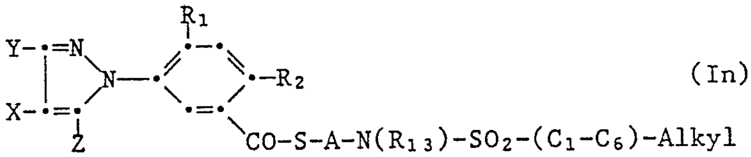

(In)

worin A, $R_1$, $R_2$, $R_{13}$, X, Y und Z die oben gegebene Bedeutung haben;
- die Verbindungen der Formel Io

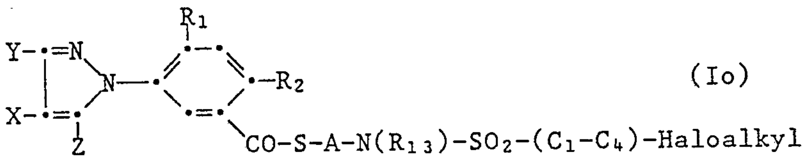

(Io)

worin A, $R_1$, $R_2$, $R_{13}$, X, Y und Z die oben gegebene Bedeutung haben;
- die Verbindungen der Formel Ip

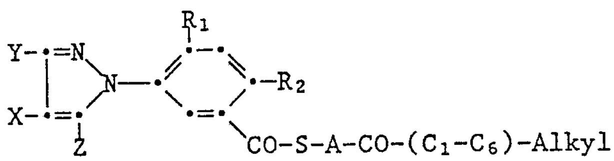

(Ip)

worin, $R_1$, $R_2$, X, Y und Z die oben gegebene Bedeutung haben;
- die Verbindungen der Formel Iq

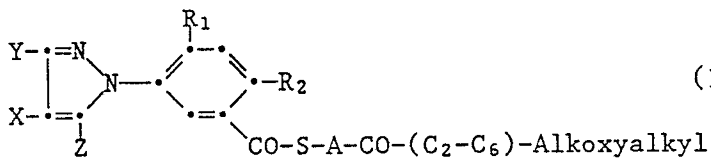

(Iq)

worin A, $R_1$, $R_2$, X, Y und Z die oben gegebene Bedeutung haben;
- die Verbindungen der Formel Ir

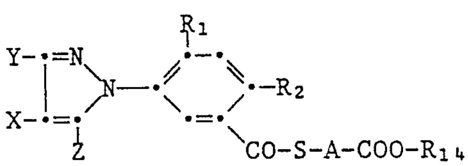

(Ir)

worin A, $R_1$, $R_2$, $R_{14}$, X, Y und Z die oben gegebene Bedeutung haben;
- die Verbindungen der Formel Is

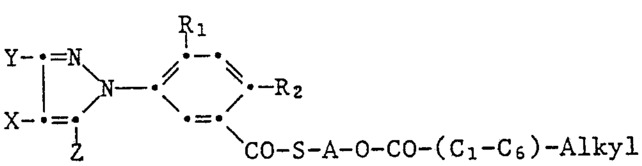

(Is)

worin A, $R_1$, $R_2$, X, Y und Z die oben gegebene Bedeutung haben;

Die Erfindung betrifft auch die möglichen Stereoisomeren, welche in Form von Enantiomeren, Diastereomeren oder deren Gemische vorliegen.

In den obigen Definitionen ist unter Halogen, Fluor, Chlor, Brom oder Iod zu verstehen, vor allem Fluor, Chlor oder Brom.

Der Ausdruck Alkyl allein oder als Teil eines Substituenten umfasst verzweigte und geradkettige Reste. Beispiele sind Methyl, Ethyl, n-Propyl, Isopropyl, n-Butyl, sec. Butyl, tert. Butyl, Isobutyl sowie die höheren Homologen, Pentyl, Hexyl, Heptyl, Octyl etc. samt ihren Isomeren. Sinngemäss enthalten Alkanoyle und Cyanalkyle ein zusätzliches Kohlenstoffatom.

Bei den in der obigen Definition erwähnten Alkenyl- oder Alkinylresten handelt es sich ebenfalls um geradkettige oder verzweigte Reste, wobei als Alkenylgruppen insbesondere 2-Propenyl (Allyl), 2-Methyl-2-propenyl (Methallyl), 1-Methyl-2-propenyl, 2-Butenyl und 3-Butenyl und als Alkinylreste 2-Propinyl (Propargyl), 2-Butinyl und 3-Butinyl zu nennen sind. Insbesondere bedeuten in Q und $R_6$ und $R_7$ die Alkenylreste Allyl, Methallyl oder 2-Butenyl und als Alkinylreste insbesondere Propargyl und 2-Butinyl. Sofern

die hier genannten ungesättigten Reste über ein Sauerstoffatom an das Molekül gebunden sind, ist der betreffende Rest vorzugsweise über ein gesättigtes Kohlenstoffatom verknüpft.

Die obige Definition von A umfasst Methylen, 1,2-Ethylen und 1,3-Propylen und die durch Substitution von 1 bis 2 Wasserstoffatomen davon abgeleiteten Gruppen, wie z.B. 1,1-Ethylen, Isopropylen (2,2-Propylen), 1,2-Propylen, 2,3-Butylen, 1,1-Dimethyl-1,2-ethylen, 1,2-Butylen, 1,3-Butylen. Von den vorgenannten Bedeutungen von A sind Methylen, 1,2-Ethylen, 1,2-Propylen und 2,3-Butylen bevorzugt.

Für die übrigen Gruppen der Formel I umfassen die angegebenen generischen Begriffe beispielsweise die folgenden spezifischen Einzelsubstituenten, wobei diese Aufzählung keine Einschränkung der Erfindung darstellt:

Halogenalkylreste können ganz oder teilweise gleich oder unterschiedlich halogensubstituiert sein, wie etwa 2,2,2-Trifluorethyl, 1,1,1,3,3,3-Hexafluorprop-2-yl, Trifluormethyl, Trichlormethyl, Difluormethyl oder Dichlormethyl.

Mit Alkoxy sind im Rahmen der jeweiligen Definitionsbreite die entsprechenden Alkoxygruppen gemeint; in erster Linie Methoxy und Ethoxy. $C_1$-$C_4$-Alkoxy-$C_2$-$C_4$-alkyl, sowie $C_1$-$C_4$-Halogenalkoxy betreffen ebenfalls im Rahmen der jeweiligen Definitionsbreite die entsprechenden Alkylradikale. Hervorzuheben sind Methoxyethyl, Ethoxyethyl, Methoxymethyl, Difluormethoxy, Dichlorfluormethoxy, Trifluormethoxy, Chlordifluormethoxy und Trichlormethoxy.

In den Substituenten, welche aus mehreren Grundelementen zusammengesetzt sind, können die Teilelemente innerhalb der Definitionsbreite frei gewählt werden.

Die Herstellung der erfindungsgemässen Pyrazole der Formel I erfolgt nach an sich bekannten Verfahren.

Die neuen 5-Pyrazol-1-yl)-benzoesäurethiolester der Formel I

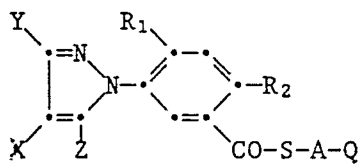

(I)

worin A, Q, $R_1$, $R_2$, X, Y und Z die oben gegebene Bedeutung hat, besteht darin, dass man eine 5-(Pyrazol-1-yl)-benzoesäure der Formel II

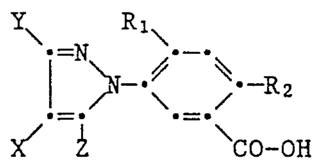

(II)

worin $R_1$, $R_2$, X, Y und Z die oben gegebene Bedeutung haben, mit einem starken Halogenierungsmittel zum Säurehalid der Formel III umwandelt,

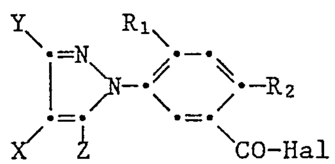

(III)

worin $R_1$, $R_2$, X, Y und Z die oben gegebene Bedeutung haben, und Hal ein Halogenatom bedeutet, dieses Säurehalid in einem inerten organischen Lösungsmittel, in Gegenwart der mindestens äquimolaren Menge einer Base mit einem Mercaptan der Formel IV

HS - A - Q (IV)

worin A und Q die oben gegebene Bedeutung haben, zum 5-(Pyrazol-1-yl)-benzoesäurethiolester der Formel I umsetzt,

oder das Benzoesäurehalid der Formel III in einem inerten organischen Lösungsmittel, in Gegenwart einer Base mit Schwefelwasserstoff behandelt und die entstandene 5-(Pyrazol-1-yl)-thiolbenzoesäure der Formel V

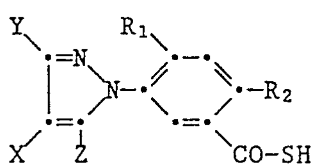

(V)

worin $R_1$, $R_2$, X, Y und Z die oben gegebene Bedeutung haben, in einem inerten organischen Lösungsmittel in Gegenwart der äquimolaren Menge einer Base mit einem Alkylhalid der Formel VI

Hal - A - Q (VI)

worin A und Q die oben gegebene Bedeutung haben und Hal ein Halogenatom, vorzugsweise Chlor oder Brom

bedeutet, zum 5-(Pyrazol-1-yl)-benzoesäurethiolester der Formel I umsetzt.

Ein weiterer Weg zur Herstellung der 5-(Pyrazol-1-yl)-benzoesäurethiolester der Formel I besteht darin, dass man eine 5-(Pyrazol-1-yl)-benzoesäure der Formel VII

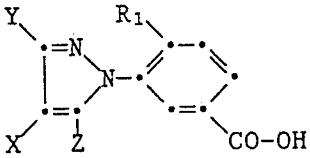

(VII)

worin $R_1$, X, Y und Z die oben gegebene Bedeutung haben, mittels eines Gemisches von konzentrierter Salpetersäure und konzentrierter Schwefelsäure zum 2-Nitro-5-(pyrazol-1-yl)-benzoesäure-Derivat der Formel VIII nitriert,

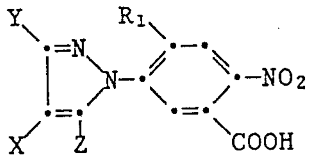

(VIII)

worin $R_1$, X, Y und Z die oben gegebene Bedeutung haben, dieses Nitrobenzoesäure-Derivat in an sich bekannter Weise mittels Wasserstoff zum 2-Amino-5-(pyrazol-1-yl)-benzoesäure-Derivat der Formel IX hydriert

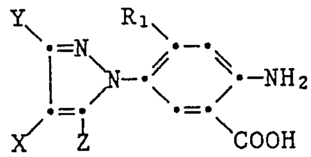

(IX)

worin $R_1$, X, Y und Z die oben gegebene Bedeutung haben, und die Aminogruppe in wässrig-saurer Lösung mittels Natriumnitrit diazotiert und mit einer Kupfer-I-Verbindung der Formel X

Cu-I-Hal (X)

worin Hal Chlor, Brom oder Iod bedeutet, zum 5-(Pyrazol-1-yl)-benzoesäure-Derivat der Formel II umsetzt, welches dann wie im vorhergehenden Verfahren mit einem starken Halogenierungsmittel zum Benzoesäurehalid der Formel III und weiterer, entweder direkt oder über die 5-(Pyrazol-1-yl)-thiolbenzoesäure der Formel V zum 5-(Pyrazol-1-yl)-benzoesäure-thiolester der Formel I umsetzt.

Als inerte Lösungs- oder Verdünnungsmittel kommen auch für diese Umsetzung höhersiedende Kohlenwasserstoffe, niedere Ester von Alkansäuren, höhersiedende Ketone und Aether in Frage. Als Beispiel seien Hexan, Benzol, Toluol, Xylol, Essigsäureethylester, Isopropyläther, Dioxan, Methylethylketon erwähnt.

Die Umsetzungen finden bei Temperaturen statt, die zwischen 0°C und dem Siedepunkt des Reaktionsgemisches liegt.

Als Basen kommen tertiäre Amine, wie Triethylamin, Pyridin oder Collidin sowie die Hydroxide, Carbonate und Bicarbonate von Alkalimetallen in Frage.

Die Reaktionsbedingungen sind bei allen beschriebenen Verfahren ähnlich.

Die Wirkstoffe der Formel I werden in der Regel mit Aufwandmengen von 0,05 bis 4 kg/ha insbesondere 0,1 bis 1 kg/ha erfolgreich eingesetzt.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchshemmende, Eigenschaften. Es werden sowohl Monokotyledonen als auch Dikotyledonen in ihrem Wachstum beeinträchtigt.

Die Wirkstoffe der Formel I können ferner zur Defoliation und Dessiccation von Baumwoll- und Kartoffelkulturen eingesetzt werden. Durch Behandeln der Kulturen im Zeitpunkt der Reife wird die Ernte der Baumwollkapseln oder der Knollen stark erleichtert, wenn die Blätter und Stauden abgefallen und/oder zur Dürre getrocknet sind.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen

Wirkstoff der Formel I enthalten, sowie Verfahren zur pre- und post-emergenten Unkrautbekämpfung und zur Hemmung des Pflanzenwuchses von monokotylen und dikotylen Pflanzen.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali-oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlensotffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffdatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze

liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual"
MC Publishing Corp., Ridgewood, New Jersey, 1979.
Dr. Helmut Stache "Tensid Taschenbuch"
Carl Hanser Verlag, München/Wien 1981.

Die herbiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

Emulgierbare Konzentrate:

| | |
|---|---|
| Wirkstoff der Formel I: | 1 bis 20 % bevorzugt 5 bis 10 % |
| oberflächenaktives Mittel: | 5 bis 30 %,vorzugsweise 10 bis 20 % |
| flüssiges Trägermittel: | 50 bis 94 %,vorzugsweise 70 bis 85 %. |

Stäube:

| | |
|---|---|
| Wirkstoff der Formel I: | 0,1 bis 10 %, vorzugsweise 0,1 bis 1 % |
| festes Trägermittel: | 99,9 bis 90 %, vorzugsweise 99,9 bis 99 %. |

Suspensions-Konzentrate:

| | |
|---|---|
| Wirkstoff der Formel I: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |

Benetzbare Pulver:

| | |
|---|---|
| Wirkstoff der Formel I: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 %. |

Granulate:

| | |
|---|---|
| Wirkstoff der Formel I: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Das nachfolgende Beispiel veranschaulicht die Herstellung einer Verbindung der Formel I. Weitere in entsprechender Weise hergestellte Wirkstoffe sind in den anschliessenden Tabellen aufgeführt. Temperaturangaben beziehen sich auf Celsius-Grade, Drucke sind in Millibar angegeben.

Beispiel 1: Herstellung von
5-[2-(3-Chlor-4,5,6,7-tetrahydro-2H-indazolyl)]-2-chlor-4-fluorbenzoesäure-(methoxycarbonylmethylthio)ester

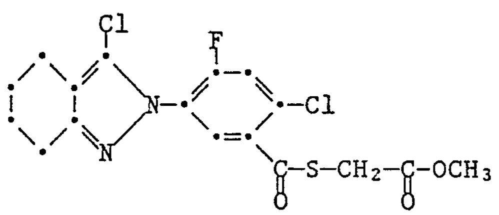

Zu einem Gemisch von 1,1 g Thioglykolsäuremethylester und 1,5 ml Triethylamin in 50 ml Toluol, tropft man unter Rühren bei Raumtemperatur, eine Lösung von 3,5 g 5-[2-(3-Chlor-4,5,6,7-tetrahydro-2H-indazolyl)]-2-chlor-4-fluorbenzoesäurechlorid in 30 ml Toluol. Nachdem alles zugetropft ist, rührt man während 3 Stunden bei Raumtemperatur weiter, filtriert dann das Triethylamin-Hydrochlorid vom Reaktionsgemisch ab und dampft das Filtrat unter Vakuum ein. Man erhält 4 g Titelprodukt mit Schmelzpunkt 98-99°C.

Analog zu diesen Beispielen werden die in den Tabellen 1-4 aufgeführten Verbindungen hergestellt.

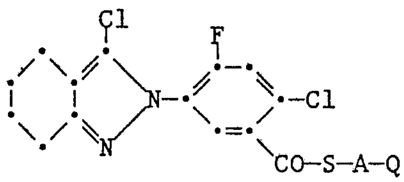

Tabelle 1

| Nr. | A-Q | phys. Daten |
|---|---|---|
| 1.001 | $-CH_2-COOH$ | |
| 1.002 | $-CH_2-COOCH_3$ | Smp. 98-99° |
| 1.003 | $-CH_2-COOC_2H_5$ | $n_D^{23}$ 1,5869 |
| 1.004 | $-CH_2-COOC_3H_7-n$ | |
| 1.005 | $-CH_2-COOCH(CH_3)_2$ | $n_D^{23}$ 1,5799 |
| 1.006 | $-CH_2-COOC_4H_9-n$ | $n_D^{23}$ 1,5748 |
| 1.007 | $-CH_2-COOCH_2CH(CH_3)_2$ | |
| 1.008 | $-CH_2-COOCH(CH_3)C_2H_5$ | |
| 1.009 | $-CH_2-COOC(CH_3)_3$ | |
| 1.010 | $-CH_2-COO(CH_2)_9CH_3$ | |
| 1.011 | $-CH_2-COOCH_2CH_2OCH_3$ | $n_D^{23}$ 1,5812 |
| 1.012 | $-CH_2-COOCH_2CH_2OC_2H_5$ | |
| 1.013 | $-CH_2-COOCH_2CH_2OC_3H_7-n$ | |
| 1.014 | $-CH_2-COO$ Cyclohexyl | $n_D^{23}$ 1,5702 |
| 1.015 | $-CH_2-COOCH_2CH_2OCH_2CH=CH_2$ | |
| 1.016 | $-CH_2-COOC(CH_3)_2CN$ | |
| 1.017 | $-CH_2COOCH_2CH_2SCH_3$ | |
| 1.018 | $-CH_2-COOCH(CH_3)CH_2N(CH_3)_2$ | |
| 1.019 | $-CH(CH_3)COOH$ | Smp. 144-146° |
| 1.020 | $-CH(CH_3)COOCH_3$ | $n_D^{20}$ 1,5855 |
| 1.021 | $-CH(CH_3)COOC_2H_5$ | $n_D^{22}$ 1,5722 |
| 1.022 | $-CH(CH_3)COOC_3H_7-n$ | |
| 1.023 | $-CH(CH_3)COOCH(CH_3)_2$ | $n_D^{22}$ 1,5705 |
| 1.024 | $-CH(CH_3)COOC_4H_9-n$ | |
| 1.025 | $-CH(CH_3)COOCH_2CH(CH_3)_2$ | |
| 1.026 | $-CH(CH_3)COOCH(CH_3)C_2H_5$ | |
| 1.027 | $-CH(CH_3)COOC(CH_3)_3$ | $n_D^{22}$ 1,5418 |

Tabelle 1 (Fortsetzung)

| Nr. | A-Q | phys. Daten |
|---|---|---|
| 1.028 | $-CH(CH_3)COO-C_5H_{11}-n$ | |
| 1.029 | $-CH(CH_3)COOCH_2CH_2OCH_3$ | |
| 1.030 | $-CH(CH_3)COOCH_2CH_2OC_2H_5$ | |
| 1.031 | $-CH(CH_3)COOCH_2CH_2OC_3H_7-n$ | |
| 1.032 | $-CH(CH_3)COOCH_2CH_2OCH_2CH=CH_2$ | |
| 1.033 | $-CH(CH_3)COOC(CH_3)_2CN$ | |
| 1.034 | $-CH(CH_3)COOCH_2CH_2SCH_3$ | |
| 1.035 | $-CH(CH_3)CH_2OH$ | |
| 1.036 | $-CH(CH_3)CN$ | |
| 1.037 | $-C_2H_4OH$ | |
| 1.038 | $-CH(CH_3)CH_2Cl$ | |
| 1.039 | $-C_2H_4Cl$ | |
| 1.040 | $-CH_2CN$ | |
| 1.041 | $-CH_2C(CH_3)_2OH$ | |
| 1.042 | $-CH_2SCN$ | |
| 1.043 | $-C_2H_4CN$ | |
| 1.044 | $-CH(CH_3)CH_2CN$ | |
| 1.045 | $-CH(CH_2OCH_3)COOCH_3$ | |
| 1.046 | $-CH_2CH(OCH_3)COOCH_3$ | |
| 1.047 | $-CH(CH_3)COOC_2H_4OCH_2CH=CH_2$ | |
| 1.048 | $-CH(CN)CH_2OCH_3$ | |
| 1.049 | $-CH_2CH(SCH_3)COOCH_3$ | |
| 1.050 | $-CH_2CH=CH_2$ | |
| 1.051 | $-CH_2CH=CHCl$ | |
| 1.052 | $-CH_2CCl=CH_2$ | |
| 1.053 | $-CH_2CH=CHCN$ | |
| 1.054 | $-CH_2C\equiv CH$ | |
| 1.055 | $-CH_2C(CH_3)=CHCOOCH_3$ | |
| 1.056 | $-C_2H_4N(CH_3)_2$ | |
| 1.057 | $-C_2H_4$-pyrrolidino | |
| 1.058 | $-C_2H_4$-piperidino | |
| 1.059 | $-C_2H_4$-morpholino | |
| 1.060 | $-CH[N(CH_3)_2]COOCH_3$ | |

Tabelle 1 (Fortsetzung)

| Nr. | A-Q | phys. Daten |
|---|---|---|
| 1.061 | $-CH(CH_3)CH_2N(CH_3)_2$ | |
| 1.062 | $-CH(CH_3)CO$-piperidino | |
| 1.063 | $-CH(CH_3)CO$-pyrrolidino | |
| 1.064 | $-CH(CH_3)CO$-4-methylpiperazino | |
| 1.065 | $-CH(CH_3)CO$-morpholino | |
| 1.066 | $-CH(CH_3)CO$-2-methylmorpholino | |
| 1.067 | $-CH(CH_3)CO$-2,6-dimethylmorpholino | |
| 1.068 | $-CH(CH_3)CO$-2-methylpiperidino | |
| 1.069 | $-CH(CH_3)CO$-3-methylpiperidino | |
| 1.070 | $-CH(CH_3)CO$-4-methylpiperidino | |
| 1.071 | $-CH(CH_3)CO-N(CH_3)CH_2CN$ | |
| 1.072 | $-CH(CH_3)CO-N(CH_2CH=CH_2)_2$ | |
| 1.073 | $-CH(CH_3)CO-OCH_2Si(CH_3)_3$ | |
| 1.074 | $-CH(CH_3)CO-OC_2H_4Si(CH_3)_3$ | |
| 1.075 | $-CH(CH_3)CO-ON=C(CH_3)_2$ | |
| 1.076 | $-CH(CH_3)CO-ON=$cyclopentyl | |
| 1.077 | $-CH(CH_3)CO-ON=$cyclohexyl | |
| 1.078 | -1,3-dioxolan-2-yleth-1-yl | |
| 1.079 | -1,3-dioxolan-2-ylmethyl | |
| 1.080 | -4-methyl-1,3-dioxolan-2-ylmethyl | |
| 1.081 | -1,3-dioxolan-2-ylpropyl | |
| 1.082 | -2-methyl-1,3-dioxolan-2-ylpropyl | |
| 1.083 | $-C_2H_4C(OCH_3)_2$ | |
| 1.084 | $-C_2H_4C(OC_2H_5)_2$ | |
| 1.085 | $-CH(CH_3)C(OC_2H_5)_2$ | |
| 1.086 | $-CH(CH_3)C(OCH_3)_2$ | |
| 1.087 | -2-methyl-1,3-dioxolan-2-ylmethyl | |
| 1.088 | $-C_2H_4Si(OC_2H_5)_3$ | |
| 1.089 | $-CH_2Si(CH_3)_3$ | $n_D^{23}$ 1,5801 |
| 1.090 | $-CH(CH_3)Si(CH_3)_3$ | |
| 1.091 | $-C_2H_4Si(CH_3)_3$ | |
| 1.092 | $-CH(CH_3)CH_2Si(CH_3)_3$ | |
| 1.093 | $-CH(CH_3)PO(OC_2H_5)_2$ | |

Tabelle 1 (Fortsetzung)

| Nr. | A-Q | phys. Daten |
|---|---|---|
| 1.094 | $-CH(CH_3)PO(CH_3)OC_2H_5$ | |
| 1.095 | $-CH(CH_3)CON(CH_3)SO_2CH_3$ | |
| 1.096 | $-CH(CH_3)CON(CH_3)SO_2CH_2Cl$ | |
| 1.097 | $-CH_2CON(CH_3)SO_2CH_3$ | |
| 1.098 | $-CH(CH_3)CON(CH_2CH=CH_2)SO_2CH_3$ | |
| 1.099 | $-CH(CH_3)CON($cyclopropyl$)SO_2CH_3$ | |
| 1.100 | $-CH(CH_3)CON[CH(CH_3)_2]SO_2CH_3$ | |
| 1.101 | $-CH_2COCH_3$ | |
| 1.102 | $-CH_2CO$ benzyl | |
| 1.103 | $-CH_2CO$ phenyl | |
| 1.104 | $-CH_2COCH_2OCH_3$ | |
| 1.105 | $-CH(CH_3)COCH_3$ | |
| 1.106 | $-CH(CH_3)COCH_2COOCH_3$ | |
| 1.107 | $-CH(COCH_3)COOC_2H_5$ | |
| 1.108 | $-CH(CH_3)COOCH(CH_3)CH_2SCH_3$ | |
| 1.109 | $-CH(CH_3)COOCH(CH_3)CH_2SC_2H_5$ | |
| 1.110 | $-CH(CH_3)COOCH(CH_3)CH_2SC_3H_7$-n | |
| 1.111 | $-CH(CH_3)COOCH(CH_3)CH_2SCH(CH_3)_2$ | |
| 1.112 | $-CH(CH_3)COOCH(CH_3)CH_2SC_4H_9$-n | |
| 1.113 | $-CH(CH_3)COOCH(CH_3)CH_2SC(CH_3)_3$ | |
| 1.114 | $-CH(CH_3)COOCH(CH_3)CH_2SCH(CH_3)C_2H_5$ | |
| 1.115 | $-CH(CH_3)CON(CH_3)CH_2CH(OCH_3)_2$ | |
| 1.116 | $-CH(CH_3)CON(CH_3)CH_2CH(OC_2H_5)_2$ | |
| 1.117 | $-CH(CH_3)COOC_2H_4OCH_2CH=CH_2$ | |
| 1.118 | $-CH(COCH_3)_2$ | |
| 1.119 | $-CH(CN)COOCH_3$ | |
| 1.120 | $-CH(OCH_3)COOCH_3$ | |
| 1.121 | $-C_2H_4N(CH_3)_2$ | $n_D^{25}$ 1,5828 |
| 1.122 | $-C_2H_4COOCH_3$ | $n_D^{25}$ 1,5655 |
| 1.123 | $-C_2H_4COOC_2H_5$ | $n_D^{25}$ 1,5615 |

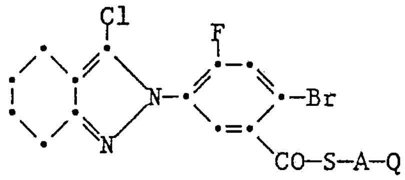

Tabelle 2

| Nr. | A-Q | phys. Daten |
|---|---|---|
| 2.001 | $-CH_2-COOH$ | |
| 2.002 | $-CH_2-COOCH_3$ | |
| 2.003 | $-CH_2-COOC_2H_5$ | |
| 2.004 | $-CH_2-COOC_3H_7-n$ | |
| 2.005 | $-CH_2-COOCH(CH_3)_2$ | |
| 2.006 | $-CH_2-COOC_4H_9-n$ | |
| 2.007 | $-CH_2-COOCH_2CH(CH_3)_2$ | |
| 2.008 | $-CH_2-COOCH(CH_3)C_2H_5$ | |
| 2.009 | $-CH_2-COOC(CH_3)_3$ | |
| 2.010 | $-CH_2-COO(CH_2)_9CH_3$ | |
| 2.011 | $-CH_2-COOCH_2CH_2OCH_3$ | |
| 4.012 | $-CH_2-COOCH_2CH_2OC_2H_5$ | |
| 2.013 | $-CH_2-COOCH_2CH_2OC_3H_7-n$ | |
| 2.014 | $-CH_2-COO$ Cyclohexyl | |
| 2.015 | $-CH_2-COOCH_2CH_2OCH_2CH=CH_2$ | |
| 2.016 | $-CH_2-COOC(CH_3)_2CN$ | |
| 2.017 | $-CH_2COOCH_2CH_2SCH_3$ | |
| 2.018 | $-CH_2-COOCH(CH_3)CH_2N(CH_3)_2$ | |
| 2.019 | $-CH(CH_3)COOH$ | |
| 2.020 | $-CH(CH_3)COOCH_3$ | |
| 2.021 | $-CH(CH_3)COOC_2H_5$ | |
| 2.022 | $-CH(CH_3)COOC_3H_7-n$ | |
| 2.023 | $-CH(CH_3)COOCH(CH_3)_2$ | |
| 2.024 | $-CH(CH_3)COOC_4H_9-n$ | |
| 2.025 | $-CH(CH_3)COOCH_2CH(CH_3)_2$ | |
| 2.026 | $-CH(CH_3)COOCH(CH_3)C_2H_5$ | |
| 2.027 | $-CH(CH_3)COOC(CH_3)_3$ | |

Tabelle 2 (Fortsetzung)

| Nr. | A-Q | phys. Daten |
|---|---|---|
| 2.028 | $-CH(CH_3)COO-C_5H_{11}-n$ | |
| 2.029 | $-CH(CH_3)COOCH_2CH_2OCH_3$ | |
| 2.030 | $-CH(CH_3)COOCH_2CH_2OC_2H_5$ | |
| 2.031 | $-CH(CH_3)COOCH_2CH_2OC_3H_7-n$ | |
| 2.032 | $-CH(CH_3)COOCH_2CH_2OCH_2CH=CH_2$ | |
| 2.033 | $-CH(CH_3)COOC(CH_3)_2CN$ | |
| 2.034 | $-CH(CH_3)COOCH_2CH_2SCH_3$ | |
| 2.035 | $-CH(CH_3)CH_2OH$ | |
| 2.036 | $-CH(CH_3)CN$ | |
| 2.037 | $-C_2H_4OH$ | |
| 2.038 | $-CH(CH_3)CH_2Cl$ | |
| 2.039 | $-C_2H_4Cl$ | |
| 2.040 | $-CH_2CN$ | |
| 2.041 | $-CH_2C(CH_3)_2OH$ | |
| 2.042 | $-CH_2SCN$ | |
| 2.043 | $-C_2H_4CN$ | |
| 2.044 | $-CH(CH_3)CH_2CN$ | |
| 2.045 | $-CH(CH_2OCH_3)COOCH_3$ | |
| 2.046 | $-CH_2CH(OCH_3)COOCH_3$ | |
| 2.047 | $-CH(CH_3)COOC_2H_4OCH_2CH=CH_2$ | |
| 2.048 | $-CH(CN)CH_2OCH_3$ | |
| 2.049 | $-CH_2CH(SCH_3)COOCH_3$ | |
| 2.050 | $-CH_2CH=CH_2$ | |
| 2.051 | $-CH_2CH=CHCl$ | |
| 2.052 | $-CH_2CCl=CH_2$ | |
| 2.053 | $-CH_2CH=CHCN$ | |
| 2.054 | $-CH_2C\equiv CH$ | |
| 2.055 | $-CH_2C(CH_3)=CHCOOCH_3$ | |
| 2.056 | $-C_2H_4N(CH_3)_2$ | |
| 2.057 | $-C_2H_4$-pyrrolidino | |
| 2.058 | $-C_2H_4$-piperidino | |
| 2.059 | $-C_2H_4$-morpholino | |
| 2.060 | $-CH[N(CH_3)_2]COOCH_3$ | |

Tabelle 2 (Fortsetzung)

| Nr. | A-Q | phys. Daten |
|---|---|---|
| 2.061 | $-CH(CH_3)CH_2N(CH_3)_2$ | |
| 2.062 | $-CH(CH_3)CO$-piperidino | |
| 2.063 | $-CH(CH_3)CO$-pyrrolidino | |
| 2.064 | $-CH(CH_3)CO$-4-methylpiperazino | |
| 2.065 | $-CH(CH_3)CO$-morpholino | |
| 2.066 | $-CH(CH_3)CO$-2-methylmorpholino | |
| 2.067 | $-CH(CH_3)CO$-2,6-dimethylmorpholino | |
| 2.068 | $-CH(CH_3)CO$-2-methylpiperidino | |
| 2.069 | $-CH(CH_3)CO$-3-methylpiperidino | |
| 2.070 | $-CH(CH_3)CO$-4-methylpiperidino | |
| 2.071 | $-CH(CH_3)CO-N(CH_3)CH_2CN$ | |
| 2.072 | $-CH(CH_3)CO-N(CH_2CH=CH_2)_2$ | |
| 2.073 | $-CH(CH_3)CO-OCH_2Si(CH_3)_3$ | |
| 2.074 | $-CH(CH_3)CO-OC_2H_4Si(CH_3)_3$ | |
| 2.075 | $-CH(CH_3)CO-ON=C(CH_3)_2$ | |
| 2.076 | $-CH(CH_3)CO-ON=$cyclopentyl | |
| 2.077 | $-CH(CH_3)CO-ON=$cyclohexyl | |
| 2.078 | -1,3-dioxolan-2-yleth-1-yl | |
| 2.079 | -1,3-dioxolan-2-ylmethyl | |
| 2.080 | -4-methyl-1,3-dioxolan-2-ylmethyl | |
| 2.081 | -1,3-dioxolan-2-ylpropyl | |
| 2.082 | -2-methyl-1,3-dioxolan-2-ylpropyl | |
| 2.083 | $-C_2H_4C(OCH_3)_2$ | |
| 2.084 | $-C_2H_4C(OC_2H_5)_2$ | |
| 2.085 | $-CH(CH_3)C(OC_2H_5)_2$ | |
| 2.086 | $-CH(CH_3)C(OCH_3)_2$ | |
| 2.087 | -2-methyl-1,3-dioxolan-2-ylmethyl | |
| 2.088 | $-C_2H_4Si(OC_2H_5)_3$ | |
| 2.089 | $-CH_2Si(CH_3)_3$ | |
| 2.090 | $-CH(CH_3)Si(CH_3)_3$ | |
| 2.091 | $-C_2H_4Si(CH_3)_3$ | |
| 2.092 | $-CH(CH_3)CH_2Si(CH_3)_3$ | |
| 2.093 | $-CH(CH_3)PO(OC_2H_5)_2$ | |

Tabelle 2 (Fortsetzung)

| Nr. | A-Q | phys. Daten |
|---|---|---|
| 2.094 | $-CH(CH_3)PO(CH_3)OC_2H_5$ | |
| 2.095 | $-CH(CH_3)CON(CH_3)SO_2CH_3$ | |
| 2.096 | $-CH(CH_3)CON(CH_3)SO_2CH_2Cl$ | |
| 2.097 | $-CH_2CON(CH_3)SO_2CH_3$ | |
| 2.098 | $-CH(CH_3)CON(CH_2CH{=}CH_2)SO_2CH_3$ | |
| 2.099 | $-CH(CH_3)CON(cyclopropyl)SO_2CH_3$ | |
| 2.100 | $-CH(CH_3)CON[CH(CH_3)_2]SO_2CH_3$ | |
| 2.101 | $-CH_2COCH_3$ | |
| 2.102 | $-CH_2CO$ benzyl | |
| 2.103 | $-CH_2CO$ phenyl | |
| 2.104 | $-CH_2COCH_2OCH_3$ | |
| 2.105 | $-CH(CH_3)COCH_3$ | |
| 2.106 | $-CH(CH_3)COCH_2COOCH_3$ | |
| 2.107 | $-CH(COCH_3)COOC_2H_5$ | |
| 2.108 | $-CH(CH_3)COOCH(CH_3)CH_2SCH_3$ | |
| 2.109 | $-CH(CH_3)COOCH(CH_3)CH_2SC_2H_5$ | |
| 2.110 | $-CH(CH_3)COOCH(CH_3)CH_2SC_3H_7$-n | |
| 2.111 | $-CH(CH_3)COOCH(CH_3)CH_2SCH(CH_3)_2$ | |
| 2.112 | $-CH(CH_3)COOCH(CH_3)CH_2SC_4H_9$-n | |
| 2.113 | $-CH(CH_3)COOCH(CH_3)CH_2SC(CH_3)_3$ | |
| 2.114 | $-CH(CH_3)COOCH(CH_3)CH_2SCH(CH_3)C_2H_5$ | |
| 2.115 | $-CH(CH_3)CON(CH_3)CH_2CH(OCH_3)_2$ | |
| 2.116 | $-CH(CH_3)CON(CH_3)CH_2CH(OC_2H_5)_2$ | |
| 2.117 | $-CH(CH_3)COOC_2H_4OCH_2CH{=}CH_2$ | |
| 2.118 | $-CH(COCH_3)_2$ | |
| 2.119 | $-CH(CN)COOCH_3$ | |
| 2.120 | $-CH(OCH_3)COOCH_3$ | |
| 2.121 | $-CH_2CH_2COOC_2H_5$ | |
| 2.122 | $-CH_2CH_2COOCH_3$ | |

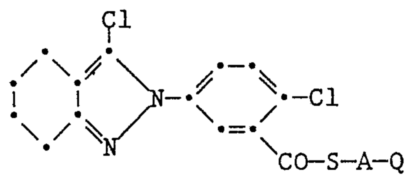

Tabelle 3

| Nr. | A-Q | phys. Daten |
|---|---|---|
| 3.001 | $-CH_2-COOH$ | |
| 3.002 | $-CH_2-COOCH_3$ | |
| 3.003 | $-CH_2-COOC_2H_5$ | |
| 3.004 | $-CH_2-COOC_3H_7-n$ | |
| 3.005 | $-CH_2-COOCH(CH_3)_2$ | |
| 3.006 | $-CH_2-COOC_4H_9-n$ | |
| 3.007 | $-CH_2-COOCH_2CH(CH_3)_2$ | |
| 3.008 | $-CH_2-COOCH(CH_3)C_2H_5$ | |
| 3.009 | $-CH_2-COOC(CH_3)_3$ | |
| 3.010 | $-CH_2-COO(CH_2)_9CH_3$ | |
| 3.011 | $-CH_2-COOCH_2CH_2OCH_3$ | |
| 3.012 | $-CH_2-COOCH_2CH_2OC_2H_5$ | |
| 3.013 | $-CH_2-COOCH_2CH_2OC_3H_7-n$ | |
| 3.014 | $-CH_2-COO$ Cyclohexyl | |
| 3.015 | $-CH_2-COOCH_2CH_2OCH_2CH=CH_2$ | |
| 3.016 | $-CH_2-COOC(CH_3)_2CN$ | |
| 3.017 | $-CH_2COOCH_2CH_2SCH_3$ | |
| 3.018 | $-CH_2-COOCH(CH_3)CH_2N(CH_3)_2$ | |
| 3.019 | $-CH(CH_3)COOH$ | |
| 3.020 | $-CH(CH_3)COOCH_3$ | |
| 3.021 | $-CH(CH_3)COOC_2H_5$ | |
| 3.022 | $-CH(CH_3)COOC_3H_7-n$ | |
| 3.023 | $-CH(CH_3)COOCH(CH_3)_2$ | |
| 3.024 | $-CH(CH_3)COOC_4H_9-n$ | |
| 3.025 | $-CH(CH_3)COOCH_2CH(CH_3)_2$ | |
| 3.026 | $-CH(CH_3)COOCH(CH_3)C_2H_5$ | |
| 3.027 | $-CH(CH_3)COOC(CH_3)_3$ | |

Tabelle 3 (Fortsetzung)

| Nr. | A-Q | phys. Daten |
|---|---|---|
| 3.028 | $-CH(CH_3)COO-C_5H_{11}-n$ | |
| 3.029 | $-CH(CH_3)COOCH_2CH_2OCH_3$ | |
| 3.030 | $-CH(CH_3)COOCH_2CH_2OC_2H_5$ | |
| 3.031 | $-CH(CH_3)COOCH_2CH_2OC_3H_7-n$ | |
| 3.032 | $-CH(CH_3)COOCH_2CH_2OCH_2CH=CH_2$ | |
| 3.033 | $-CH(CH_3)COOC(CH_3)_2CN$ | |
| 3.034 | $-CH(CH_3)COOCH_2CH_2SCH_3$ | |
| 3.035 | $-CH(CH_3)CH_2OH$ | |
| 3.036 | $-CH(CH_3)CN$ | |
| 3.037 | $-C_2H_4OH$ | |
| 3.038 | $-CH(CH_3)CH_2Cl$ | |
| 3.039 | $-C_2H_4Cl$ | |
| 3.040 | $-CH_2CN$ | |
| 3.041 | $-CH_2C(CH_3)_2OH$ | |
| 3.042 | $-CH_2SCN$ | |
| 3.043 | $-C_2H_4CN$ | |
| 3.044 | $-CH(CH_3)CH_2CN$ | |
| 3.045 | $-CH(CH_2OCH_3)COOCH_3$ | |
| 3.046 | $-CH_2CH(OCH_3)COOCH_3$ | |
| 3.047 | $-CH(CH_3)COOC_2H_4OCH_2CH=CH_2$ | |
| 3.048 | $-CH(CN)CH_2OCH_3$ | |
| 3.049 | $-CH_2CH(SCH_3)COOCH_3$ | |
| 3.050 | $-CH_2CH=CH_2$ | |
| 3.051 | $-CH_2CH=CHCl$ | |
| 3.052 | $-CH_2CCl=CH_2$ | |
| 3.053 | $-CH_2CH=CHCN$ | |
| 3.054 | $-CH_2C{\equiv}CH$ | |
| 3.055 | $-CH_2C(CH_3)=CHCOOCH_3$ | |
| 3.056 | $-C_2H_4N(CH_3)_2$ | |
| 3.057 | $-C_2H_4$-pyrrolidino | |
| 3.058 | $-C_2H_4$-piperidino | |
| 3.059 | $-C_2H_4$-morpholino | |
| 3.060 | $-CH[N(CH_3)_2]COOCH_3$ | |

Tabelle 3 (Fortsetzung)

| Nr. | A-Q | phys. Daten |
|---|---|---|
| 3.061 | $-CH(CH_3)CH_2N(CH_3)_2$ | |
| 3.062 | $-CH(CH_3)CO$-piperidino | |
| 3.063 | $-CH(CH_3)CO$-pyrrolidino | |
| 3.064 | $-CH(CH_3)CO$-4-methylpiperazino | |
| 3.065 | $-CH(CH_3)CO$-morpholino | |
| 3.066 | $-CH(CH_3)CO$-2-methylmorpholino | |
| 3.067 | $-CH(CH_3)CO$-2,6-dimethylmorpholino | |
| 3.068 | $-CH(CH_3)CO$-2-methylpiperidino | |
| 3.069 | $-CH(CH_3)CO$-3-methylpiperidino | |
| 3.070 | $-CH(CH_3)CO$-4-methylpiperidino | |
| 3.071 | $-CH(CH_3)CO-N(CH_3)CH_2CN$ | |
| 3.072 | $-CH(CH_3)CO-N(CH_2CH=CH_2)_2$ | |
| 3.073 | $-CH(CH_3)CO-OCH_2Si(CH_3)_3$ | |
| 3.074 | $-CH(CH_3)CO-OC_2H_4Si(CH_3)_3$ | |
| 3.075 | $-CH(CH_3)CO-ON=C(CH_3)_2$ | |
| 3.076 | $-CH(CH_3)CO-ON=$cyclopentyl | |
| 3.077 | $-CH(CH_3)CO-ON=$cyclohexyl | |
| 3.078 | -1,3-dioxolan-2-yleth-1-yl | |
| 3.079 | -1,3-dioxolan-2-ylmethyl | |
| 3.080 | -4-methyl-1,3-dioxolan-2-ylmethyl | |
| 3.081 | -1,3-dioxolan-2-ylpropyl | |
| 3.082 | -2-methyl-1,3-dioxolan-2-ylpropyl | |
| 3.083 | $-C_2H_4C(OCH_3)_2$ | |
| 3.084 | $-C_2H_4C(OC_2H_5)_2$ | |
| 3.085 | $-CH(CH_3)C(OC_2H_5)_2$ | |
| 3.086 | $-CH(CH_3)C(OCH_3)_2$ | |
| 3.087 | -2-methyl-1,3-dioxolan-2-ylmethyl | |
| 3.088 | $-C_2H_4Si(OC_2H_5)_3$ | |
| 3.089 | $-CH_2Si(CH_3)_3$ | |
| 3.090 | $-CH(CH_3)Si(CH_3)_3$ | |
| 3.091 | $-C_2H_4Si(CH_3)_3$ | |
| 3.092 | $-CH(CH_3)CH_2Si(CH_3)_3$ | |
| 3.093 | $-CH(CH_3)PO(OC_2H_5)_2$ | |

Tabelle 3 (Fortsetzung)

| Nr. | A-Q | phys. Daten |
|---|---|---|
| 3.094 | $-CH(CH_3)PO(CH_3)OC_2H_5$ | |
| 3.095 | $-CH(CH_3)CON(CH_3)SO_2CH_3$ | |
| 3.096 | $-CH(CH_3)CON(CH_3)SO_2CH_2Cl$ | |
| 3.097 | $-CH_2CON(CH_3)SO_2CH_3$ | |
| 3.098 | $-CH(CH_3)CON(CH_2CH{=}CH_2)SO_2CH_3$ | |
| 3.099 | $-CH(CH_3)CON(\text{cyclopropyl})SO_2CH_3$ | |
| 3.100 | $-CH(CH_3)CON[CH(CH_3)_2]SO_2CH_3$ | |
| 3.101 | $-CH_2COCH_3$ | |
| 3.102 | $-CH_2CO$ benzyl | |
| 3.103 | $-CH_2CO$ phenyl | |
| 3.104 | $-CH_2COCH_2OCH_3$ | |
| 3.105 | $-CH(CH_3)COCH_3$ | |
| 3.106 | $-CH(CH_3)COCH_2COOCH_3$ | |
| 3.107 | $-CH(COCH_3)COOC_2H_5$ | |
| 3.108 | $-CH(CH_3)COOCH(CH_3)CH_2SCH_3$ | |
| 3.109 | $-CH(CH_3)COOCH(CH_3)CH_2SC_2H_5$ | |
| 3.110 | $-CH(CH_3)COOCH(CH_3)CH_2SC_3H_7\text{-n}$ | |
| 3.111 | $-CH(CH_3)COOCH(CH_3)CH_2SCH(CH_3)_2$ | |
| 3.112 | $-CH(CH_3)COOCH(CH_3)CH_2SC_4H_9\text{-n}$ | |
| 3.113 | $-CH(CH_3)COOCH(CH_3)CH_2SC(CH_3)_3$ | |
| 3.114 | $-CH(CH_3)COOCH(CH_3)CH_2SCH(CH_3)C_2H_5$ | |
| 3.115 | $-CH(CH_3)CON(CH_3)CH_2CH(OCH_3)_2$ | |
| 3.116 | $-CH(CH_3)CON(CH_3)CH_2CH(OC_2H_5)_2$ | |
| 3.117 | $-CH(CH_3)COOC_2H_4OCH_2CH{=}CH_2$ | |
| 3.118 | $-CH(COCH_3)_2$ | |
| 3.119 | $-CH(CN)COOCH_3$ | |
| 3.120 | $-CH(OCH_3)COOCH_3$ | |

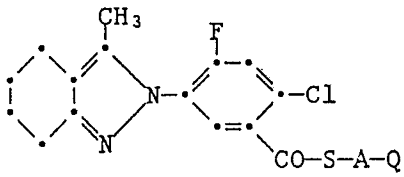

Tabelle 4

| Nr. | A-Q | phys. Daten |
|---|---|---|
| 4.001 | $-CH_2-COOH$ | |
| 4.002 | $-CH_2-COOCH_3$ | $n_D^{24}$ 1,5832 |
| 4.003 | $-CH_2-COOC_2H_5$ | $n_D^{25}$ 1,5795 |
| 4.004 | $-CH_2-COOC_3H_7-n$ | |
| 4.005 | $-CH_2-COOCH(CH_3)_2$ | |
| 4.006 | $-CH_2-COOC_4H_9-n$ | |
| 4.007 | $-CH_2-COOCH_2CH(CH_3)_2$ | |
| 4.008 | $-CH_2-COOCH(CH_3)C_2H_5$ | |
| 4.009 | $-CH_2-COOC(CH_3)_3$ | |
| 4.010 | $-CH_2-COO(CH_2)_9CH_3$ | |
| 4.011 | $-CH_2-COOCH_2CH_2OCH_3$ | $n_D^{25}$ 1,5578 |
| 4.012 | $-CH_2-COOCH_2CH_2OC_2H_5$ | |
| 4.013 | $-CH_2-COOCH_2CH_2OC_3H_7-n$ | |
| 4.014 | $-CH_2-COO$ Cyclohexyl | |
| 4.015 | $-CH_2-COOCH_2CH_2OCH_2CH=CH_2$ | |
| 4.016 | $-CH_2-COOC(CH_3)_2CN$ | |
| 4.017 | $-CH_2COOCH_2CH_2SCH_3$ | |
| 4.018 | $-CH_2-COOCH(CH_3)CH_2N(CH_3)_2$ | |
| 4.019 | $-CH(CH_3)COOH$ | |
| 4.020 | $-CH(CH_3)COOCH_3$ | $n_D^{24}$ 1,5749 |
| 4.021 | $-CH(CH_3)COOC_2H_5$ | $n_D^{26}$ 1,5428 |
| 4.022 | $-CH(CH_3)COOC_3H_7-n$ | $n_D^{21}$ 1,5491 |
| 4.023 | $-CH(CH_3)COOCH(CH_3)_2$ | $n_D^{26}$ 1,5394 |
| 4.024 | $-CH(CH_3)COOC_4H_9-n$ | |
| 4.025 | $-CH(CH_3)COOCH_2CH(CH_3)_2$ | |
| 4.026 | $-CH(CH_3)COOCH(CH_3)C_2H_5$ | |
| 4.027 | $-CH(CH_3)COOC(CH_3)_3$ | |

Tabelle 4 (Fortsetzung)

| Nr. | A-Q | phys. Daten |
|---|---|---|
| 4.028 | $-CH(CH_3)COO-C_5H_{11}-n$ | |
| 4.029 | $-CH(CH_3)COOCH_2CH_2OCH_3$ | |
| 4.030 | $-CH(CH_3)COOCH_2CH_2OC_2H_5$ | |
| 4.031 | $-CH(CH_3)COOCH_2CH_2OC_3H_7-n$ | |
| 4.032 | $-CH(CH_3)COOCH_2CH_2OCH_2CH=CH_2$ | |
| 4.033 | $-CH(CH_3)COOC(CH_3)_2CN$ | |
| 4.034 | $-CH(CH_3)COOCH_2CH_2SCH_3$ | |
| 4.035 | $-CH(CH_3)CH_2OH$ | |
| 4.036 | $-CH(CH_3)CN$ | |
| 4.037 | $-C_2H_4OH$ | |
| 4.038 | $-CH(CH_3)CH_2Cl$ | |
| 4.039 | $-C_2H_4Cl$ | |
| 4.040 | $-CH_2CN$ | |
| 4.041 | $-CH_2C(CH_3)_2OH$ | |
| 4.042 | $-CH_2SCN$ | |
| 4.043 | $-C_2H_4CN$ | |
| 4.044 | $-CH(CH_3)CH_2CN$ | |
| 4.045 | $-CH(CH_2OCH_3)COOCH_3$ | |
| 4.046 | $-CH_2CH(OCH_3)COOCH_3$ | |
| 4.047 | $-CH(CH_3)COOC_2H_4OCH_2CH=CH_2$ | |
| 4.048 | $-CH(CN)CH_2OCH_3$ | |
| 4.049 | $-CH_2CH(SCH_3)COOCH_3$ | |
| 4.050 | $-CH_2CH=CH_2$ | |
| 4.051 | $-CH_2CH=CHCl$ | |
| 4.052 | $-CH_2CCl=CH_2$ | |
| 4.053 | $-CH_2CH=CHCN$ | |
| 4.054 | $-CH_2C\equiv CH$ | |
| 4.055 | $-CH_2C(CH_3)=CHCOOCH_3$ | |
| 4.056 | $-C_2H_4N(CH_3)_2$ | |
| 4.057 | $-C_2H_4$-pyrrolidino | |
| 4.058 | $-C_2H_4$-piperidino | |
| 4.059 | $-C_2H_4$-morpholino | |
| 4.060 | $-CH[N(CH_3)_2]COOCH_3$ | |

Tabelle 4 (Fortsetzung)

| Nr. | A-Q | phys. Daten |
|---|---|---|
| 4.061 | $-CH(CH_3)CH_2N(CH_3)_2$ | |
| 4.062 | $-CH(CH_3)CO$-piperidino | |
| 4.063 | $-CH(CH_3)CO$-pyrrolidino | |
| 4.064 | $-CH(CH_3)CO$-4-methylpiperazino | |
| 4.065 | $-CH(CH_3)CO$-morpholino | |
| 4.066 | $-CH(CH_3)CO$-2-methylmorpholino | |
| 4.067 | $-CH(CH_3)CO$-2,6-dimethylmorpholino | |
| 4.068 | $-CH(CH_3)CO$-2-methylpiperidino | |
| 4.069 | $-CH(CH_3)CO$-3-methylpiperidino | |
| 4.070 | $-CH(CH_3)CO$-4-methylpiperidino | |
| 4.071 | $-CH(CH_3)CO-N(CH_3)CH_2CN$ | |
| 4.072 | $-CH(CH_3)CO-N(CH_2CH=CH_2)_2$ | |
| 4.073 | $-CH(CH_3)CO-OCH_2Si(CH_3)_3$ | |
| 4.074 | $-CH(CH_3)CO-OC_2H_4Si(CH_3)_3$ | |
| 4.075 | $-CH(CH_3)CO-ON=C(CH_3)_2$ | |
| 4.076 | $-CH(CH_3)CO-ON=$cyclopentyl | |
| 4.077 | $-CH(CH_3)CO-ON=$cyclohexyl | |
| 4.078 | -1,3-dioxolan-2-yleth-1-yl | |
| 4.079 | -1,3-dioxolan-2-ylmethyl | |
| 4.080 | -4-methyl-1,3-dioxolan-2-ylmethyl | |
| 4.081 | -1,3-dioxolan-2-ylpropyl | |
| 4.082 | -2-methyl-1,3-dioxolan-2-ylpropyl | |
| 4.083 | $-C_2H_4C(OCH_3)_2$ | |
| 4.084 | $-C_2H_4C(OC_2H_5)_2$ | |
| 4.085 | $-CH(CH_3)C(OC_2H_5)_2$ | |
| 4.086 | $-CH(CH_3)C(OCH_3)_2$ | |
| 4.087 | -2-methyl-1,3-dioxolan-2-ylmethyl | |
| 4.088 | $-C_2H_4Si(OC_2H_5)_3$ | |
| 4.089 | $-CH_2Si(CH_3)_3$ | |
| 4.090 | $-CH(CH_3)Si(CH_3)_3$ | |
| 4.091 | $-C_2H_4Si(CH_3)_3$ | |
| 4.092 | $-CH(CH_3)CH_2Si(CH_3)_3$ | |
| 4.093 | $-CH(CH_3)PO(OC_2H_5)_2$ | |

Tabelle 4 (Fortsetzung)

| Nr. | A-Q | phys. Daten |
|---|---|---|
| 4.094 | $-CH(CH_3)PO(CH_3)OC_2H_5$ | |
| 4.095 | $-CH(CH_3)CON(CH_3)SO_2CH_3$ | |
| 4.096 | $-CH(CH_3)CON(CH_3)SO_2CH_2Cl$ | |
| 4.097 | $-CH_2CON(CH_3)SO_2CH_3$ | |
| 4.098 | $-CH(CH_3)CON(CH_2CH=CH_2)SO_2CH_3$ | |
| 4.099 | $-CH(CH_3)CON(cyclopropyl)SO_2CH_3$ | |
| 4.100 | $-CH(CH_3)CON[CH(CH_3)_2]SO_2CH_3$ | |
| 4.101 | $-CH_2COCH_3$ | |
| 4.102 | $-CH_2CO$ benzyl | |
| 4.103 | $-CH_2CO$ phenyl | |
| 4.104 | $-CH_2COCH_2OCH_3$ | |
| 4.105 | $-CH(CH_3)COCH_3$ | |
| 4.106 | $-CH(CH_3)COCH_2COOCH_3$ | |
| 4.107 | $-CH(COCH_3)COOC_2H_5$ | |
| 4.108 | $-CH(CH_3)COOCH(CH_3)CH_2SCH_3$ | |
| 4.109 | $-CH(CH_3)COOCH(CH_3)CH_2SC_2H_5$ | |
| 4.110 | $-CH(CH_3)COOCH(CH_3)CH_2SC_3H_7-n$ | |
| 4.111 | $-CH(CH_3)COOCH(CH_3)CH_2SCH(CH_3)_2$ | |
| 4.112 | $-CH(CH_3)COOCH(CH_3)CH_2SC_4H_9-n$ | |
| 4.113 | $-CH(CH_3)COOCH(CH_3)CH_2SC(CH_3)_3$ | |
| 4.114 | $-CH(CH_3)COOCH(CH_3)CH_2SCH(CH_3)C_2H_5$ | |
| 4.115 | $-CH(CH_3)CON(CH_3)CH_2CH(OCH_3)_2$ | |
| 4.116 | $-CH(CH_3)CON(CH_3)CH_2CH(OC_2H_5)_2$ | |
| 4.117 | $-CH(CH_3)COOC_2H_4OCH_2CH=CH_2$ | |
| 4.118 | $-CH(COCH_3)_2$ | |
| 4.119 | $-CH(CN)COOCH_3$ | |
| 4.120 | $-CH(OCH_3)COOCH_3$ | |

Formulierungsbeispiele:

Beispiel 2: Formulierungsbeispiele für Wirkstoffe der Formel I (% = Gewichtsprozent)

| a) Spritzpulver | a) | b) | c) |
|---|---|---|---|
| Wirkstoff gemäss Tabellen 1 bis 4 | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff gemäss Tabellen 1 bis 4 | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff gemäss Tabellen 1 bis 4 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühlen vermahlen wird.

| d) Extruder Granulat | a) | b) |
|---|---|---|
| Wirkstoff gemäss Tabellen 1 bis 4 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff gemäss Tabellen 1 bis 4 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff gemäss Tabellen 1 bis 4 | 40 % | 5 % |
| Aethylenglykol | 10 % | 10 % |
| Nonylphenolpolyäthylen-glykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75%igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspsionen jeder gewünschten Konzentration hergestellt werden können.

| g) Salzlösung | |
|---|---|
| Wirkstoff gemäss Tabellen 1 bis 4 | 5 % |
| Isopropylamin | 1 % |
| Octylphenolpolyäthylenglykoläther (78 Mol AeO) | 3 % |
| Wasser | 91 % |

Biologische Beispiele:

Beispiel 3: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25 %igen Emulsions- konzentrat behandelt. Es werden Konzentrationsreihen von 60 bis 4000 g Wirksubstanz/Hektar angewendet. Der Saatschalen werden im Gewächshaus bei 22-25° C und 50-70 % relativer Luftfeuchtigkeit gehalten und der Versuch nach 3 Wochen ausgewertet. Der Zustand der Pflanzen wurde gemäss folgender Notenskala boniliert

| | |
|---|---|
| 1 | Pflanze hat nicht gekeimt oder ist eingegangen |
| 2-3 | sehr starke Schäden |
| 4 | starke Schäden |
| 5 | mittlere Schäden, die Pflanzen sind verkümmert |
| 6 | Schäden, welche die Pflanze regenerieren kann |
| 7-8 | leichte Schäden |
| 9 | normales Wachstum, wie das unbehandelter Pflanzen |

In diesem Versuch zeigten die geprüften Verbindungen der Tabellen 1 bis 4 starke Herbizidwirkung. Die Resultate der Verbindung 1.027 sind zusammengefasst in der Tabelle 5.

Tabelle 5:

| Pflanze | 2000 g/ha | 1000 g/ha | 300 g/ha | 250 g/ha | 125 g/ha |
|---|---|---|---|---|---|
| Gerste | 9 | 9 | 9 | 9 | 9 |
| Weizen | 8 | 9 | 9 | 9 | 9 |
| Mais | 7 | 8 | 9 | 9 | 9 |
| Hirse | 7 | 7 | 9 | 9 | 9 |
| Soja | 9 | 9 | 9 | 9 | 9 |
| Sonnenblume | 7 | 8 | 9 | 9 | 9 |
| Abutilon | 1 | 1 | 4 | 4 | 5 |
| Sida spinosa | 1 | 1 | 4 | 4 | 5 |
| Amaranthus sp. | 1 | 1 | 1 | 1 | 1 |
| Chenopodium sp. | 1 | 1 | 1 | 1 | 1 |
| Solanum nigrum | 1 | 1 | 1 | 1 | 1 |
| Chrysanthemum leuc. | 1 | 1 | 1 | 1 | 1 |
| Viola tricolor | 1 | 1 | 1 | 1 | 1 |
| Veronica sp. | 1 | 1 | 1 | 1 | 1 |

Beispiel 4: Post-emergente Herbizid-Wirkung (Selektivwirkung in Getreide)

Eine Anzahl Kulturpflanzen und Unkräuter wurden nach dem Auflaufen (im 4-bis 6-Blattstadium) mit einer wässrigen Wirkstoffdispersion in Dosierungen von 60-2000 g Wirksubstanz pro Hektar auf die Pflanzen gespritzt und diese bei 24°-26°C und 45-60 % relativer Luftfeuchtigkeit gehalten. 15 Tage nach der Behandlung wird der Versuch nach der obigen Notenskala ausgewertet. Die geprüften Verbindungen der Tabellen 1-4 zeigten in diesem Versuch starke Herbizidwirkung, einige Resultate sind in den Tabellen 6 und 7 zusammengefasst.

Tabelle 6: Aufwandmenge 1000 g/ha

| Pflanze | Verbindung No. 1.002 | 1.003 | 1.005 | 1.006 | 1.011 | 1.014 | 1.020 |
|---|---|---|---|---|---|---|---|
| Gerste | 6 | 6 | 7 | 8 | 6 | 8 | 7 |
| Weizen | 7 | 7 | 8 | 8 | 7 | 9 | 7 |
| Abutilon | 1 | 1 | 1 | 1 | 1 | 1 | 1 |
| Sida spinosa | 1 | 2 | 1 | 1 | 4 | 4 | 1 |
| Chenopodium sp. | 2 | 3 | 1 | 1 | 2 | 4 | 1 |
| Solanum nigrum | 1 | 1 | 1 | 1 | 1 | 1 | 1 |
| Ipomoea purp. | 1 | 1 | 1 | 1 | 1 | 1 | 1 |
| Sinapis album | 1 | 1 | 1 | 2 | 1 | 3 | 1 |
| Chrysanthemum leuc. | 1 | 1 | 1 | 1 | 1 | 1 | 1 |
| Galium aparine | 3 | 1 | 1 | 4 | 4 | 2 | 2 |
| Viola tricolor | 3 | 1 | 1 | 1 | 1 | 1 | 1 |
| Veronica sp. | 1 | 1 | 1 | 1 | 1 | 1 | 1 |

Tabelle 6: (Fortsetzung)

| Pflanze | Verbindung No. 1.021 | 1.023 | 1.027 | 1.122 | 1.123 | 4.021 |
|---|---|---|---|---|---|---|
| Gerste | 7 | 6 | 8 | 7 | 6 | 6 |
| Weizen | 6 | 7 | 8 | 7 | 6 | 7 |
| Abutilon | 1 | 1 | 1 | 1 | 1 | 1 |
| Sida spinosa | 1 | 1 | 4 | 1 | 1 | 1 |
| Chenopodium sp. | 1 | 1 | 2 | 1 | 1 | 1 |
| Solanum nigrum | 1 | 1 | 1 | 1 | 1 | 1 |
| Ipomoea purp. | 1 | 1 | 2 | 1 | 1 | 1 |
| Sinapis album | 1 | 1 | 1 | 1 | 1 | 4 |
| Chrysanthemum leuc. | 1 | 1 | 1 | 1 | 1 | 1 |
| Galium aparine | 2 | 1 | 3 | 4 | 4 | 4 |
| Viola tricolor | 2 | 1 | 1 | 1 | 1 | 1 |
| Veronica sp. | 1 | 4 | 3 | 1 | 1 | 1 |

Tabelle 7: Aufwandmenge 250 g/ha

| Pflanze | Verbindung No. 1.002 | 1.003 | 1.005 | 1.006 | 1.011 | 1.014 | 1.020 |
|---|---|---|---|---|---|---|---|
| Gerste | 8 | 8 | 8 | 9 | 8 | 8 | 8 |
| Weizen | 8 | 9 | 9 | 9 | 9 | 9 | 8 |
| Mais | 6 | 6 | 8 | 7 | 6 | 7 | 6 |
| Abutilon | 2 | 1 | 1 | 1 | 1 | 1 | 1 |
| Sida spinosa | 3 | 3 | 4 | 4 | 4 | 4 | 3 |
| Chenopodium sp. | 3 | 4 | 4 | 4 | 3 | 4 | 2 |
| Solanum nigrum | 1 | 1 | 2 | 1 | 1 | 1 | 1 |
| Ipomoea purp. | 1 | 1 | 1 | 1 | 1 | 1 | 1 |
| Sinapis album | 3 | 2 | 1 | 2 | 3 | 3 | 5 |
| Chrysanthemum leuc. | 2 | 1 | 1 | 2 | 1 | 1 | 2 |
| Galium aparine | 3 | 2 | 4 | 4 | 4 | 4 | 2 |
| Viola tricolor | 4 | 1 | 1 | 1 | 3 | 1 | 4 |
| Veronica sp. | 2 | 1 | 2 | 3 | 1 | 4 | 1 |

Tabelle 7: (Fortsetzung)

| Pflanze | Verbindung No. 1.021 | 1.023 | 1.027 | 1.122 | 1.123 | 4.021 |
|---|---|---|---|---|---|---|
| Gerste | 8 | 8 | 8 | 8 | 7 | 7 |
| Weizen | 8 | 9 | 9 | 8 | 8 | 8 |
| Mais | 7 | 7 | 8 | 6 | 6 | 6 |
| Abutilon | 1 | 1 | 3 | 1 | 1 | 1 |
| Sida spinosa | 3 | 4 | 5 | 1 | 1 | 1 |
| Chenopodium sp. | 3 | 1 | 3 | 4 | 1 | 1 |
| Solanum nigrum | 1 | 1 | 1 | 1 | 1 | 1 |
| Ipomoea purp. | 1 | 1 | 2 | 1 | 1 | 1 |
| Sinapis album | 4 | 2 | 1 | 1 | 2 | 4 |
| Chrysanthemum leuc. | 2 | 2 | 1 | 1 | 1 | 1 |
| Galium aparine | 2 | 4 | 4 | 4 | 4 | 4 |
| Viola tricolor | 3 | 3 | 1 | 1 | 1 | 1 |
| Veronica sp. | 2 | 4 | 4 | 1 | 1 | 1 |

Beispiel 5: Selektive Herbizidwirkung in Soja

Sojapflanzen sowie Gräser und Unkräuter werden im Gewächshaus angezogen bis sie nach ca. 2 Wochen das 4-Blatt Stadium erreicht haben. Dann werden sie mit einer verdünnten Wirkstoffbrühe gespritzt. Die behandelten Pflanzen werden im Gewächshaus bei optimalen Wuchsbedingungen, 26-28° C, 43-60 % relative Luftfeuchtigkeit, regelmässiges Bewässern, gehalten. Der Versuch wird 21 Tage nach der Behandlung ausgewertet und der Zustand der Pflanzen gemäss der obigen Notenskala ausgewertet. Die geprüften Verbindungen der Tabellen 1 bis 4 waren Soja gegenüber tolerant, schädigten aber die mitgeprüften Unkräuter. Einige Resultate sind in den Tabellen 8 und 9 zusammengefasst.

Tabelle 8

Aufwandmenge 500 g/ha

| Pflanze | Verbindung No. 1.002 | 1.003 | 1.011 | 1.020 | 1.021 | 1.023 | 1.122 | 4.021 |
|---|---|---|---|---|---|---|---|---|
| Soja | 8 | 6 | 7 | 7 | 7 | 6 | 6 | 6 |
| Sorghum sp. | 4 | 4 | 4 | 3 | 3 | 3 | 4 | 3 |
| Lolium perenne | 4 | 4 | 5 | 4 | 4 | 3 | 3 | 5 |
| Echinochloa | 4 | 4 | 4 | 5 | 5 | 3 | 4 | 4 |
| Abutilon | 1 | 1 | 1 | 1 | 1 | 1 | 1 | 1 |
| Sida spinosa | 3 | 2 | 4 | 1 | 2 | 1 | 1 | 1 |
| Xanthium | 3 | 1 | 1 | 2 | 4 | 3 | 3 | 4 |
| Amaranthus ret. | 2 | 4 | 4 | 2 | 2 | 1 | 1 | 4 |
| Chenopodium sp. | 2 | 3 | 2 | 2 | 1 | 1 | 1 | 1 |
| Solanum nigrum | 1 | 1 | 1 | 1 | 1 | 1 | 1 | 1 |
| Ipomoea purp. | 1 | 1 | 1 | 1 | 1 | 1 | 1 | 1 |
| Sinapis album | 1 | 2 | 2 | 4 | 3 | 1 | 1 | 4 |
| Chrysanthemum leuc. | 1 | 1 | 1 | 2 | 1 | 1 | 1 | 1 |
| Galium aparine | 3 | 2 | 4 | 2 | 2 | 4 | 3 | 4 |
| Viola tricolor | 3 | 1 | 1 | 2 | 2 | 1 | 1 | 2 |
| Veronica sp. | 1 | 1 | 1 | 1 | 2 | 1 | 1 | 4 |

Tabelle 9

Aufwandmenge 125 g/ha

| Pflanze | Verbindung No. 1.003 | 1.005 | 1.006 | 1.011 | 1.014 | 1.023 | 1.122 | 1.123 | 4.021 |
|---|---|---|---|---|---|---|---|---|---|
| Soja | 7 | 8 | 8 | 7 | 7 | 8 | 7 | 7 | 7 |
| Abutilon | 1 | 1 | 1 | 1 | 1 | 1 | 1 | 1 | 1 |
| Xanthium | 1 | 2 | 2 | 1 | 3 | 4 | 4 | 4 | 4 |
| Solanum nigrum | 1 | 2 | 2 | 1 | 2 | 1 | 3 | 1 | 1 |
| Ipomoea purp. | 1 | 1 | 1 | 1 | 2 | 1 | 1 | 1 | 1 |
| Chrysan-themum leuc. | 1 | 1 | 3 | 2 | 2 | 2 | 1 | 1 | 1 |
| Galium aparine | 2 | 4 | 4 | 4 | 4 | 4 | 4 | 4 | 4 |
| Viola tricolor | 1 | 2 | 1 | 3 | 2 | 4 | 1 | 1 | 2 |
| Veronica sp. | 1 | 4 | 4 | 3 | 4 | 4 | 1 | 1 | 4 |

Beispiel 6: Herbizidwirkung vor dem Auflaufen der Pflanzen

Kunststofftöpfe werden mit expandiertem Vermiculit (Dichte: 0,135 g/$cm^3$, Wasserabsorptionsvermögen: 0,565 l/l) gefüllt. Nach dem Sättigen des nicht adsorptiven Vermiculits mit einer wässrigen Wirkstoffemulsion in deinonisiertem Wasser, die die Wirkstoffe in einer Konzentration von 70,8 ppm enthält, werden Samen der folgenden Pflanzen auf die Oberfläche gesät. Nasturtium offcinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen bewertet. Die geprüften Verbindungen der Tabellen 1 bis 4 zeigen dabei gute bis sehr gute Herbizidwirkung.

Beispiel 7 Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe. Die daher verwendete Dosis entspricht einer Wirkstoffmenge von 0,5 kg Wirkstoff pro Hektar (Spritzbrühmenge = 550 l/ha. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt. Der Zustand der Pflanzen wird nach oben gegebenen Notenskala verwertet. Die geprüften Verbindungen der Tabellen 1 bis 4 zeigten in diesem Test gute Wirkung gegen die beiden Unkräuter.

Beispiel 8: Desiccations- und Defoliationswirkung

In einem Gewächshaus wurden Baumwollpflanzen der Sorte Deltapine in Tontöpfen angezogen. Nach abgeschlossener Kapselbildung wurden sie mit wässerigen Zubereitungen des Wirkstoffes No. 1 in einer Aufwandmenge, die 1,2, 0,6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Dessikation (% Austrocknung der an der Pflanze verbleibenden Blätter).

In diesem Versuch beliessen die geprüften Verbindungen der Tabellen 1 bis 4 bei Aufwandmengen von 0,6 und 1,2 kg/ha nach 7 Tagen bloss noch wenige vertrocknete Blätter auf den Pflanzen (> 80 % Blattfall und Austrocknung).

Beispiel 9: Wuchshemmung bei Gräsern mit Klee

Eine Mischung der Gräser Poa, Festuca, Lolium, Bromus, Cynosurus und Klee (Trifolium/pratense/repens) wird in 15 cm-Kunststofftöpfen mit steriler Landerde angesät und im Gewächshaus bei einer Tagestemperatur

von 21° C und einer Nachttemperatur von 17° C angezogen. Die Beleuchtungsdauer ist 13,5 Stunden/Tag bei einer Lichtintensität von mindestens 7000 Lux. Nach dem Auflauf werden die Pflanzen wöchentlich auf ca. 6 cm Höhe zurückgeschnitten. Ca. 42 Tage nach der Saat und 1 Tag nach dem letzten Schnitt erfolgt die Applikation mit 0,3 bis 3 kg Wirkstoff/ha, welcher als Spritzpulver formuliert ist und mit einer Wasseraufwandmenge von 500 l/ha. 3 Wochen nach der Behandlung findet die Auswertung statt. Hierbei wird die Höhe des Neuzuwachses gemessen und in % zum Durchschnitt der unbehandelten Kontrollen dargestellt.

Die geprüften Verbindungen der Tabellen 1-4 bewirken eine Reduzierung des Neuzuwachses um 10 bis 80 %.

## Patentansprüche

1. 5-Pyrazol-1-yl-benzoesäurethiolester der Formel I

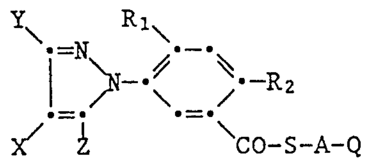

(I)

worin

X Wasserstoff oder Halogen

Y $C_1$-$C_4$-Alkyl oder

X und Y gemeinsam mit den Kohlenstoffatomen an die sie gebunden sind einen 5-6-gliedrigen Ring bilden, der unsubstituiert oder durch Methyl substituiert ist,

Z Halogen, $C_1$-$C_6$-Alkyl, $C_1$-$C_4$-Alkoxy oder $C_1$-$C_4$-Alkylthio

$R_1$ Wasserstoff oder Halogen,

$R_2$ Halogen,

A-Q gemeinsam Wasserstoff oder ein Rest $-CH(COCH_3)COOR_4$,

A eine geradkettige oder verzweigte $C_1$-$C_4$-Alkylenbrücke, die unsubstituiert oder ein- oder mehrfach durch $C_1$-$C_4$-Alkoxy, $C_1$-$C_4$-Alkylthio oder -CN substituiert ist,

Q Hydroxyl, Halogen, -CN, -SCN, $C_2$-$C_6$-Alkenyl, $C_2$-$C_6$-Haloalkenyl, $C_2$-$C_6$-Cyanoalkenyl, $C_2$-$C_4$-Alkinyl oder einen Rest $-C(R_3)=CH-COOR_4$, $-CH[N(R_3)_2]COOR_3$, $-NR_5R_6$, $-CONR_7R_8$, $-Si(R_{11})_3$, $-COOCH_2Si(CH_3)_2C_1$-$C_6$-alkyl, $-COON=C(R_9)_2$, $-C(R_3)=(OR_{10})_2$, $-PO(OR_{12})$-$(O)_pR_{12}$, $-CON(R_{13})SO_2$-$C_1$-$C_6$-alkyl, $-CON(R_{13})$-$SO_2C_1$-$C_4$-Haloalkyl, $C_1$-$C_6$-Alkylcarbonyl, $C_2$-$C_6$-Alkoxyalkylcarbonyl einen Benzoyl-, Benzylcarbonylrest, der unsubstituiert oder durch Halogen, $C_1$-$C_4$-Alkyl, $C_1$-$C_4$-Haloalkyl, $C_1$-$C_4$-Alkoxy, $C_1$-$C_4$-Haloalkoxy, Cyan oder Nitro ein- oder mehrfach substituiert ist, ferner einen Rest $COOR_{14}$, $-CON(R_3)CH_2C(OC_1$-$C_6$-Alkyl$)_2$ oder $C_1$-$C_6$-Alkanoyloxyrest,

p ist Null oder 1,

$R_3$ Wasserstoff, $C_1$-$C_6$-Alkyl oder $C_2$-$C_6$-Alkoxyalkyl,

$R_4$ $C_1$-$C_6$-Alkyl oder $C_1$-$C_6$-Hydroxyalkyl,

$R_5$ und $R_6$ unabhängig voneinander je Wasserstoff, $C_1$-$C_6$-Alkyl, $C_2$-$C_6$-Alkoxyalkyl, $C_3$-$C_6$-Alkenyl, $C_3$-$C_6$-Alkinyl oder $C_3$-$C_6$-Cycloalkyl, 2-Furanylmethyl, 2-Tetrahydrofuranylmethyl, 2-(5-Methyl)-tetrahydrofuranylmethyl, 2-Thienylmethyl,

$R_5$ und $R_6$ zusammen mit dem Stickstoffatom an das sie gebunden sind, den Pyrrolidino-, Piperidino- oder Morpholinorest,

$R_7$ und $R_8$ unabhängig voneinander je Wasserstoff, $C_1$-$C_{12}$-Alkyl, $C_3$-$C_8$-Alkenyl, $C_3$-$C_6$-Alkinyl, $C_2$-$C_8$-Alkoxyalkyl, $C_1$-$C_4$-Alkoxy, Benzyl, Phenyl oder $C_1$-$C_4$-Cyanoalkyl,

$R_7$ und $R_8$ zusammen mit dem Stickstoffatom, an das sie gebunden sind, einen Pyrrolidino-, Piperidino-, Morpholino-, Thiomorpholino-, 4-Methylpiperazino-, Pyrazolino-, Imidazolino- oder 1,2,4-Triazolinrest der unsubstituiert oder ein- oder zweifach durch $C_1$-$C_4$-Alkyl substituiert ist,

$R_9$ unabhängig voneinander je $C_1$-$C_6$-Alkyl oder zusammen $C_3$-$C_7$-Cycloalkyl,

$R_{10}$ unabhängig voneinander $C_1$-$C_4$-Alkyl oder $C_1$-$C_4$-Haloalkyl oder zusammen eine 1,2-Aethylen-, 1,3-Propylen- oder 1,2-Cyclohexylenbrücke,

$R_{11}$ unabgängig voneinander $C_1$-$C_6$-Alkyl oder $C_1$-$C_6$-Alkoxy,

$R_{12}$ unabhängig voneinander Wasserstoff, $C_1$-$C_6$-Alkyl, $C_1$-$C_6$-Haloalkyl, $C_1$-$C_6$-Cyanoalkyl, $C_3$-$C_4$-Alkenyl oder $C_3$-$C_4$-Alkinyl,

$R_{13}$ $C_1$-$C_4$-Alkyl oder $C_3$-$C_7$-Cycloalkyl,

$R_{14}$ Wasserstoff, $C_1$-$C_{12}$-Alkyl, $C_1$-$C_6$-Alkoxy-$C_1$-$C_4$-alkyl, $C_3$-$C_7$-Alkenyl, $C_3$-$C_6$-Alkinyl, $C_3$-$C_7$-Cycloalkyl, $C_3$-$C_5$-Alkenyloxy-$C_1$-$C_4$-alkyl, $C_2$-$C_6$-Alkylthio-$C_1$-$C_4$-alkyl oder $C_1$-$C_4$-Dialkylamino-$C_1$-$C_4$-alkyl bedeuten.

2. 2-[2-(4,5,6,7-tetrahydro-2H-indazolyl)]-benzoesäurethiolester gemäss Anspruch 1 der Formel

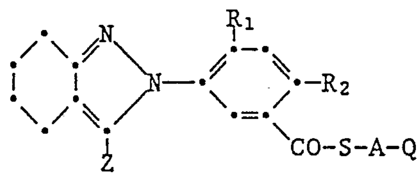

worin $R_1$, Fluor, $R_2$ Chlor oder Brom, A eine $C_1$-$C_4$-Alkylenbrücke, Z Chlor oder Methyl bedeuten und Q die im Anspruch 1 gegebene Bedeutung hat.

3. Ein 5-[2-(4,5,6,7-Tetrahydro-2H-indazolyl)]-benzoesäurethiolester gemäss Anspruch 2 der Formel

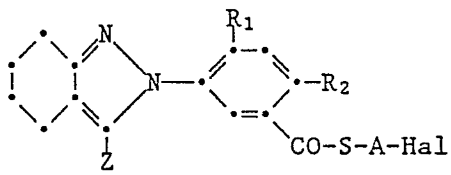

worin A, $R_1$, $R_2$ und Z die im Anspruch 1 gegebene Bedeutung haben und Hal ein Halogenatom bedeutet.

4. Ein 5-[2-(4,5,6,7-Tetrahydro-2H-indazolyl)]-benzoesäurethiolester gemäss Anspruch 2 der Formel

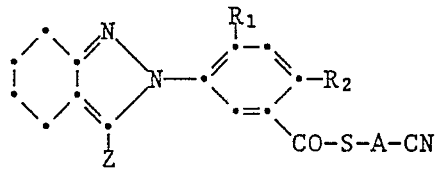

worin A, $R_1$, $R_2$ und Z die im Anspruch 1 gegebene Bedeutung haben.

5. Ein 5-[2-(4,5,6,7-Tetrahydro-2H-indazolyl)]-benzoesäurethiolester gemäss Anspruch 2 der Formel

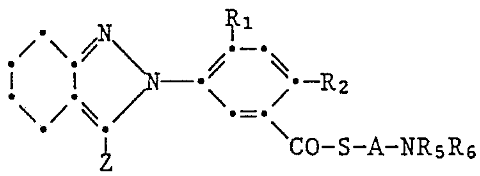

worin A, $R_1$, $R_2$, $R_5$, $R_6$ und Z die im Anspruch 1 gegebene Bedeutung haben.

6. 5-[2-(3-Chlor-4,5,6,7-tetrahydro-2H-indazolyl)]-2-chlor-4-fluorbenzoesäure-(N,N-dimethylamino-ethylthio)ester gemäss Anspruch 5.

7. Ein 5-[2-(4,5,6,7-Tetrahydro-2H-indazolyl)]-benzoesäurethiolester gemäss Anspruch 2 der Formel

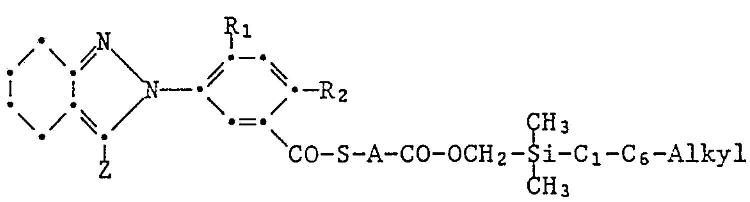

worin A, $R_1$, $R_2$ und Z die oben gegebene Bedeutung haben.

8. 5-[2-(3-Chlor-4,5,6,7-tetrahydro-2H-indazolyl)]-2-chlor-4-fluorbenzoesäure-(trimethylsilylmethylthio)ester gemäss Anspruch 7.

9. Ein 3-[2-(4,5,6,7-Tetrahydro-2H-indazolyl)]-benzoesäurethiolester gemäss Anspruch 2 der Formel

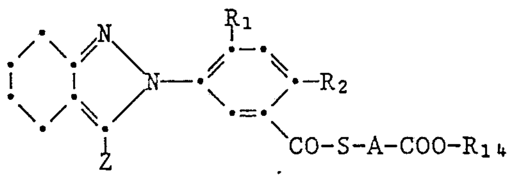

worin A, $R_1$, $R_2$ und Z die im Anspruch 1 gegebene Bedeutung haben.

10. Ein 5-[2-(4,5,6,7-tetrahydro-2H-indazolyl)]-benzoesäure-thiolester gemäss Anspruch 9 ausgewählt aus

5-[2-(3-Chlor-4,5,6,7-tetrahydro-2H-indazolyl)]-2-chlor-4-fluorbenzoesäure-(methoxycarbonylmethylthio)ester,
5-[2-(3-Chlor-4,5,6,7-tetrahydro-2H-indazolyl)]-2-chlor-4-fluorbenzoesäure-(methoxycarbonyl-eth-1-ylthio)ester,
5-[2-(3-Chlor-4,5,6,7-tetrahydro-2H-indazolyl)]-2-chlor-4-fluorbenzoesäure-(ethoxycarbonyl-eth-1-ylthio)ester,
5-[2-(3-Chlor-4,5,6,7-tetrahydro-2H-indazolyl)]-2-chlor-4-fluorbenzoesäure-(isopropoxycarbonyl-eth-1-ylthio)ester,
5-[2-(3-Methyl-4,5,6,7-tetrahydro-2H-indazolyl)]-2-chlor-4-fluorbenzoesäure-(methoxycarbonylmethylthio)ester,
5-[2-(3-Methyl-4,5,6,7-tetrahydro-2H-indazolyl)]-2-chlor-4-fluorbenzoesäure-(ethoxycarbonylmethylthio)ester,
5-[2-(3-Methyl-4,5,6,7-tetrahydro-2H-indazolyl)]-2-chlor-4-fluorbenzoesäure-(isopropoxycarbonyl-eth-1-ylthio)ester,
5-[2-(3-Methyl-4,5,6,7-tetrahydro-2H-indazolyl)]-2-chlor-4-fluorbenzoesäure-(methoxycarbonyl-eth-1-ylthio)ester,
5-[2-(3-Methyl-4,5,6,7-tetrahydro-2H-indazolyl)]-2-chlor-4-fluorbenzoesäure-(ethoxycarbonyl-eth-1-ylthio)ester,
5-[2-(3-Methyl-4,5,6,7-tetrahydro-2H-indazolyl)]-2-chlor-4-fluorbenzoesäure-(n-propoxycarbonyl-eth-1-ylthio)ester,
5-[2-(3-Methyl-4,5,6,7-tetrahydro-2H-indazolyl)]-2-chlor-4-fluorbenzoesäure-(methoxyethoxycarbonyl-methylthio)ester,
5-[2-(3-Chlor-4,5,6,7-tetrahydro-2H-indazolyl)]-2-chlor-4-fluorbenzoesäure-(methoxycarbonyl-ethylthio)ester,
5-[2-(3-Chlor-4,5,6,7-tetrahydro-2H-indazolyl)]-2-chlor-4-fluorbenzoesäure-(ethoxycarbonyl-ethylthio)ester,
5-[2-(3-Chlor-4,5,6,7-tetrahydro-2H-indazolyl)]-2-chlor-4-fluorbenzoesäure-(carbonyl-eth-1-ylthio)ester,
5-[2-(3-Chlor-4,5,6,7-tetrahydro-2H-indazolyl)]-2-chlor-4-fluorbenzoesäure-(ethoxycarbonyl-methylthio)ester,
5-[2-(3-Chlor-4,5,6,7-tetrahydro-2H-indazolyl)]-2-chlor-4-fluorbenzoesäure-(isopropoxycarbonyl-methylthio)ester,
5-[2-(3-Chlor-4,5,6,7-tetrahydro-2H-indazolyl)]-2-chlor-4-fluorbenzoesäure-(n-butoxycarbonyl-methylthio)ester,
5-[2-(3-Chlor-4,5,6,7-tetrahydro-2H-indazolyl)]-2-chlor-4-fluorbenzoesäure-(methoxyethoxycarbonyl-methylthio)ester,
5-[2-(3-Chlor-4,5,6,7-tetrahydro-2H-indazolyl)]-2-chlor-4-fluorbenzoesäure-(tert.butoxycarbonyl-eth-1-ylthio)ester,
5-[2-(3-Chlor-4,5,6,7-tetrahydro-2H-indazolyl)]-2-chlor-4-fluorbenzoesäure-(cyclohexyloxycarbonyl-methylthio)ester.

11. Verfahren zur Herstellung der 5-(Pyrazol-1-yl)-benzoesäurethiolester der Formel I, Anspruch 1, dadurch gekennzeichnet, dass man ein 5-Pyrazol-1-yl-benzoesäurehalid der Formel III

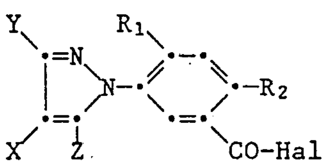

(III)

worin $R_1$, $R_2$, X, Y und Z die im Anspruch 1 gegebene Bedeutung haben und Hal ein Halogenatom, vorzugsweise Chlor oder Brom bedeutet in einem inerten organischen Lösungsmittel in Gegenwart der mindestens äquimolaren Menge einer anorganischen Base mit einem Mercaptan der Formel IV,

HS - A - Q (IV)

worin A und Q die oben gegebene Bedeutung haben, umsetzt zum 5-Imido-benzoesäurethiolester der Formel I oder das Säurehalid der Formel III in einem inerten organischen Lösungsmittel, in Gegenwart einer Base mit Schwefelwasserstoff behandelt und die entstandene 5-Pyrazol-1-yl-thiolbenzoesäure der Formel V

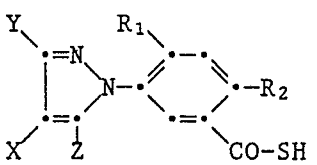

(V)

worin $R_1$, $R_2$, X, Y und Z die im Anspruch 1 gegebene Bedeutung haben in an sich bekannter Weise in einem inerten organischen Lösungsmittel, in Gegenwart der äquimolaren Menge einer Base mit einem

Alkylhalid der Formel IV
Hal -A - Q (IV)
worin A und Q die im Anspruch 1 gegebene Bedeutung haben und Hal ein Halogenatom, vorzugsweise Chlor oder Brom bedeutet, zum 1-Imido-benzoesäurethiolester der Formel I umsetzt.

12. Ein herbizides und Pflanzenwachstum regulierendes Mittel, dadurch gekennzeichnet, dass es neben Träger- und/oder anderen Zuschlagstoffen als Wirkstoff ein 5-Pyrazol-1-yl-benzoesäurethiolester der Formel I, Anspruch 1 enthält.

13. Verfahren zur selektiven pre- oder postemergenten Bekämpfung von Unkräutern in Nutzpflanzenkulturen, dadurch gekennzeichnet, dass man die Nutzpflanzenkulturen oder deren Anbaufläche mit einer wirksamen Menge eines Wirkstoffes der Formel I, Anspruch 1 oder eines eine solche Verbindung enthaltenden Mittels behandelt.

14. Verfahren zur Defoliation und Dessiccation von Baumwoll- und Kartoffelkulturen zum Erleichtern der Ernte, dadurch gekennzeichnet, dass man die Kultur kurz vor der Ernte mit einer wirksamen Menge eines Wirkstoffes gemäss Anspruch 1 oder eines eine solche Verbindung enthaltendes Mittels behandelt.